# EUROPEAN PATENT SPECIFICATION

(11) **EP 3 346 968 B1**
(45) Date of publication and mention of the grant of the patent: **09.09.2026**
(21) Application number: 16845192.0
(22) Date of filing: 09.09.2016
(51) Int. Cl.: A01N 1/146, A61M 1/02, B01D 71/70, B01D 71/24, B01D 69/08, B01D 69/06, B01D 69/02, B01D 67/00, A61J 1/14, A61J 1/10, B01D 63/08, B01D 69/10

(54) **OXYGEN DEPLETION DEVICE FOR DEPLETING OXYGEN FROM BLOOD PRIOR TO ANAEROBIC STORAGE**
SAUERSTOFFABREICHERUNGSVORRICHTUNG ZUM ABREICHERN VON SAUERSTOFF AUS BLUT VOR EINER ANAEROBEN LAGERUNG
DISPOSITIF D'APPAUVRISSEMENT EN OXYGÈNE PERMETTANT D'APPAUVRIR L'OXYGÈNE DU SANG AVANT LE STOCKAGE ANAÉROBIE

(30) Priority: 10.09.2015 US 201562216774 P; 08.09.2016 US 201662385116 P
(43) Date of publication of application: 18.07.2018
(73) Proprietor: Hemanext Inc., Lexington, MA 02421 (US)
(72) Inventor: HARHEN, Robert, Bethesda, Maryland 20817 (US); PIGNONE, Peter, Bethesda, Maryland 20817 (US); RENGANATHAN, Narendran, Bethesda, Maryland 20817 (US); SARITA, Jancarlo, Bethesda, Maryland 20817 (US); SUTTON, Jeffrey, Bethesda, Maryland 20817 (US); WOLF, Michael, Bethesda, Maryland 20817 (US); ZOCCHI, Michael, Bethesda, Maryland 20817 (US)
(74) Representative: Bassil, Nicholas Charles
(86) International application number: PCT/US2016/051115
(87) International publication number: WO 2017/044856

(56) References cited:
- EP-A1- 3 285 711
- WO-A1-2012/027582
- US-A- 4 093 515
- US-A- 4 162 676
- US-A- 4 314 480
- US-A- 4 837 047
- US-A- 5 449 617
- US-A- 6 162 396
- US-A- 6 162 396
- US-B1- 6 287 284
- US-B2- 6 494 909
- US-B2- 8 535 421
- E. CONVERSE II PEIRCE ET AL: "THE MEMBRANE LUNG: STUDIES WITH A NEW HIGH PERMEABILITY CO-POLYMER MEMBRANE", TRANSACTIONS - AMERICAN SOCIETY FOR ARTIFICIAL INTERNAL ORGANS, vol. 14, no. 1, 1 April 1968 (1968-04-01), pages 220 - 226, XP055576917

## Description

### FIELD OF THE INVENTION

The present disclosure relates to Oxygen Reduction Disposable kits (ORDKit), devices and methods for the improved preservation of whole blood and blood components. More particularly, the disclosure relates to the improved devices and methods for the collection of blood and blood components to provide whole blood and blood components having reduced levels of oxygen. The methods, devices and kits of the present disclosure provide for improved quality of blood and blood components for transfusion and improved patient safety and outcome.

### BACKGROUND OF THE INVENTION

WO 2016/145210 A1 and WO 2016/172645 A1 disclose devices and methods having collapsible blood containers **102** (and collapsible blood containers **202)** made of silicone.

Gas permeable blood bags made with thin (20-50 µm) silicone sheets are structurally weak. To ensure rapid gas depletion during processing, blood containers for oxygen depletion are designed to be thin, leading to structural weakness. Because blood bags must survive rough handling, bags must be structurally robust while still maintaining gas permeability. Blood bags need to be able to survive impacts without leaking or rupturing when dropped. Blood bags further need to be puncture and tear resistant.

Typical methods used in the past to reinforce gas permeable materials utilize rigid frames or support structures, such as perforated metal or plastic, which add cost and also detract from the pliability of the membrane. Such approaches tend to be incompatible with existing blood collection methodologies and require significantly more storage space than the accepted collapsed blood collection kits.

Suitable thin, silicone membranes for use in blood gas depletion devices are not commercially available. In the past, typical methods used to reinforce silicone sheets utilized embedded glass fibers, which detract from the gas permeability. Previous silicone sheets used to prepare reinforced sheets were 150 µm or greater in thickness. While both are more resistant to structural failure and also easier to reinforce, such thick reinforced sheets did not provide the desired oxygen reduction rates. Preparing silicone sheets and collapsible blood containers having silicone thicknesses ranging from 14 µm to 100 µm posed challenges including bursting when handling, unwanted adhesion and cohesion, and puncturing.

Further, ISO 3826-1:2013 requires that plastic collapsible blood containers shall not show leakage when placed between two plates and subjected to an internal pressure of 50 kPa above atmospheric pressure for 10 minutes. To solve these problems, gas permeable sheets of silicone sheet have been reinforced with plastic mesh or fabrics to provide the necessary structural integrity for the gas permeable collapsible blood containers. Such reinforced silicone sheets provide for the preparation of collapsible blood containers that when filled with liquid can survive drops of up to 6 feet and are resistant to tearing and punctures.

US Patent No. 6,162,396 discloses a blood storage device for removal of oxygen from blood, comprising: an oxygen-impermeable outer layer; an oxygen-permeable, red blood cell compatible inner layer contained within the outer layer; and an oxygen scrubber material placed between the outer layer and the inner layer.

### SUMMARY OF THE INVENTION

The present invention provides an oxygen depletion device as defined in claim 1. Particular embodiments are defined in the dependent claims.

### BRIEF DESCRIPTION OF THE DRAWINGS

Some aspects of the disclosure are herein described, by way of example only, with reference to the accompanying drawings. With specific reference now to the drawings in detail, it is stressed that the particulars shown are by way of example and are for purposes of illustrative discussion of embodiments of the disclosure. In this regard, the description, taken with the drawings, makes apparent to those skilled in the art how aspects of the disclosure may be practiced.

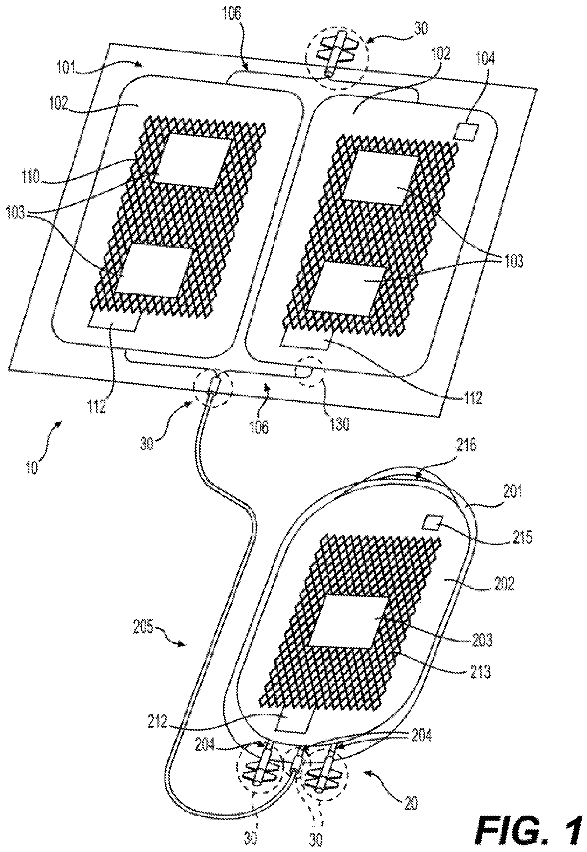

Figure 1 illustrates an exemplary embodiment of an oxygen reduction disposable storage system having a blood depletion device having two reinforced silicone collapsible blood containers **102,** respectively, and an anaerobic storage bag having a reinforced silicone collapsible blood container **202** according to the present disclosure.
Figure 2 illustrates exemplary structures of reinforced silicone membranes **600** according to the present disclosure.
Figure 3 illustrates an exemplary embodiment of a reinforced silicone collapsible blood container **102** according to the present disclosure having a reinforced silicone membrane **600** or a reinforced silicone membrane **700.**
Figure 4 illustrates an exemplary embodiment of a reinforced silicone collapsible blood container **102** according to the present disclosure having a reinforced silicone membrane **600** or a reinforced silicone membrane **700.**
Figure 5 illustrates an exemplary embodiment of a reinforced silicone collapsible blood container **102** according to the present disclosure having a reinforced silicone membrane **600** or a reinforced silicone membrane **700.**
Figure 6 illustrates a reinforced silicone membrane **700** having a raised feature **701,** an open area **702,** a thickness **703,** and having a pattern **722** according to the present disclosure.
Figure 7A to 7I illustrates exemplary embodiments of cross sections of raised features **701** according to the present disclosure having a height of length **714** and a width of length **713.**
Figure 8A to 8N illustrates exemplary patterns **720** of raised features **701** of a reinforced silicone membrane **700** according to the present disclosure.
Figure 9 illustrates an exemplary embodiment of a reinforced silicone collapsible blood container **102** prepared using an injection molding process comprising a reinforced silicone membrane **700** having a raised feature **701,** an open area **702,** a thickness **703,** and having a pattern **724** according to the present disclosure.

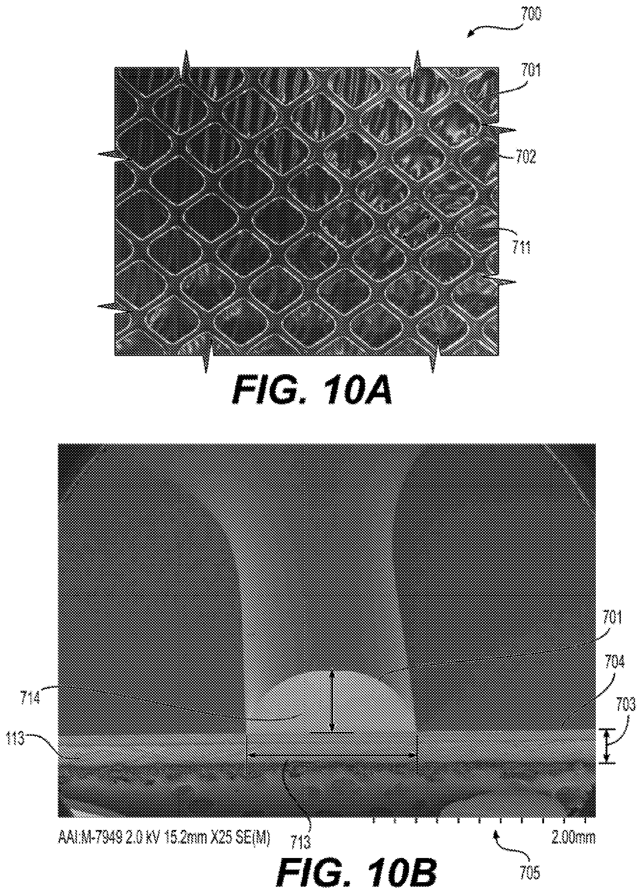

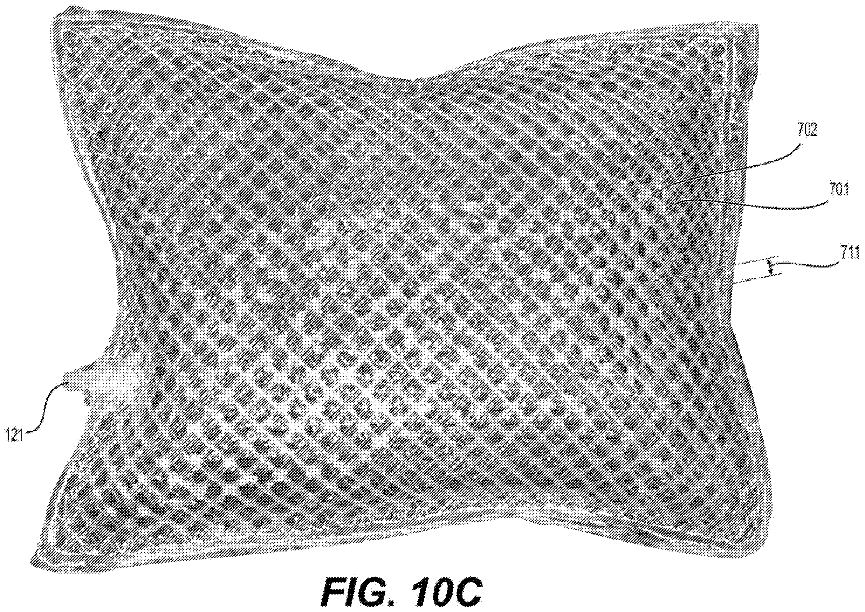

Figure 10 illustrates an exemplary embodiment of a reinforced silicone membrane **700** and a collapsible blood container **202** prepared using a reinforced silicone membrane **700.** Figure 10A shows a reinforced silicone membrane **700** having a raised feature **701** having a pattern **721,** and an open area **702.** Figure 10B shows a reinforced silicone membrane **700** of Figure 10A having a cross section **761,** a height of length **714,** and a width of length **713.** Figure 10C presents a collapsible blood container **102** prepared from a reinforced silicone membrane **700** of Figure 10A.
Figures 11A and 11B illustrate exemplary embodiments of a reinforced silicone membrane **700** prepared by compression molding and by injection molding respectively according to the present disclosure.

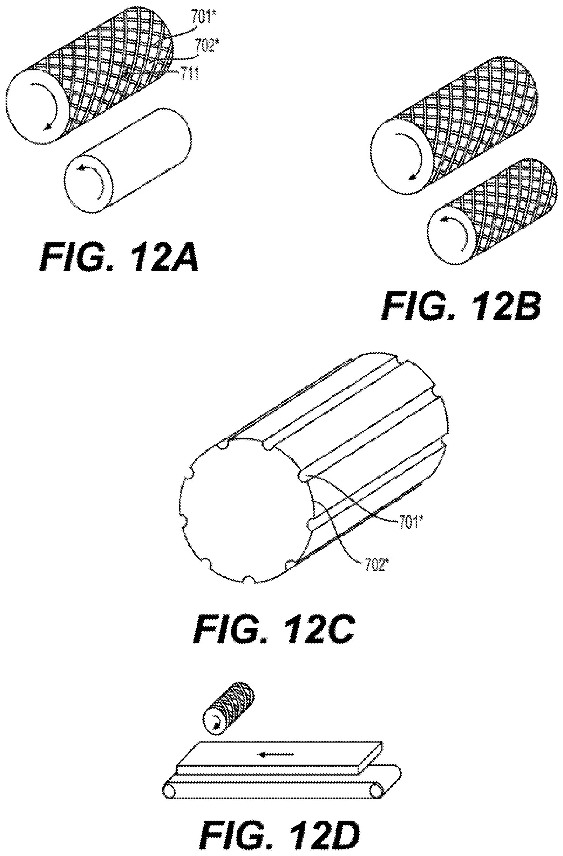

Figure 12A to 12D illustrate exemplary embodiments of roller dies suitable for use in the manufacture of a reinforced silicone membrane **700** using a calendaring method according to an aspect of the present disclosure.
Figure 13 illustrates an exemplary embodiment of an automated method of manufacturing reinforced silicone collapsible blood containers according to the present disclosure.
Figure 14 illustrates an exemplary embodiment of an automated method of manufacturing reinforced silicone collapsible blood containers using a continuous process according to the present disclosure.

Corresponding reference characters indicate corresponding parts throughout the several views. The examples set out herein illustrate several embodiments of the invention but should not be construed as limiting the scope of the invention as defined in the appended claims.

In this specification the following non-SI units are used, which may be converted to the respective SI or metric unit according to the following conversion table:

| *Name of unit* | *Conversion factor* | *SI or metric unit* |
|---|---|---|
| *inch* | *2.54* | *cm* |

### DETAILED DESCRIPTION

The present disclosure includes and provides devices and methodology for the preservation of blood and blood components in which the preparation of oxygen reduced blood and blood components is initiated at the donor collection stage.

As used herein, the term "bag" refers to collapsible containers prepared from a flexible material and includes pouches, tubes, and gusset bags. As used herein, the term includes folded bags having one, two, three, or more folds and which are sealed or bonded on one, two, three, or more sides. Bags may be prepared using a variety of techniques known in the art including bonding of sheets of one or more materials. Methods of bonding materials to form bags are known in the art. Also included and provided for in the present disclosure are containers prepared by injection and blow molding. Methods to prepare blow molded and injection molded containers are known in the art. Preferred types of blow molded or injection molded containers are flexible containers that can be reduced in size for efficient packing and shipping while being capable of expanding to accommodate blood or blood components for reduction of oxygen. They also may be designed to conform to the volume of the blood until they are fully expanded. As used throughout the present disclosure, the bags are a form of collapsible container and the two terms are used interchangeably throughout the present disclosure.

As used herein, the term "collapsible container" includes bags, containers, enclosures, envelopes, pouches, pockets, receptacles, and other devices that can contain and retain a liquid or fluid. In certain aspects, the collapsible container may be manufactured by conventional means such as injection molding or insert molding. In other aspects, the collapsible container may be prepared from sheets of polymer materials that are bonded together using methods known in the art to prepare containers capable of holding a volume. Such collapsible containers are well known in the art. See, for example, U.S. Patent 3,942,529 issued to Waage; U.S. Patent 4,131,200 issued to Rinfret; and U.S. Patent 5,382,526 issued to Gajewski et al. Suitable methods for bonding polymer materials to prepare collapsible containers according to the present disclosure include heat welding, ultrasonic welding, radio frequency (RF) welding, and solvent welding. In certain aspects, multiple bonding methods may be used to prepare collapsible containers according to the present disclosure. Collapsible container according to the present disclosure include enclosures having one or more pleats, folds, diaphragms, bubbles, and gussets. Methods for preparing collapsible containers are known in the art. See, for example, U.S. Patent 3,361,041 issued to Grob; U.S. Patent 4,731,978 issued to Martensson; U.S. Patent 4,998,990 issued to Richter et al.; and U.S. Patent 4,262,581 issued to Ferrell. Also included and provided for in the present disclosure are containers having combinations of both flexible and inflexible parts, wherein the flexible parts allow for the expansion of the volume through, for example, pleats, folds or gussets and other similar geometric features in the packaging shape, whereas the inflexible parts may provide rigidity and geometry definition to the container. Methods and designs for preparing collapsible containers having both flexible and inflexible parts are known in the art, such as described by Randall in U.S. Patent 6,164,821 and by LaFleur in U.S. Patent 5,328,268.

As used herein the term "about" refers to ± 10 %.

The terms "comprises," "comprising," "includes," "including," "having," and their conjugates mean "including but not limited to."

The term "consisting of" means "including and limited to."

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular forms "a," "an," and "the" include plural references unless the context clearly dictates otherwise. For example, the term "a compound" or "at least one compound" may include a plurality of compounds, including mixtures thereof.

The present disclosure provides for, and includes, an oxygen depletion device **10** for depleting oxygen from blood comprising an outer receptacle **101** substantially impermeable to oxygen, inner collapsible blood container **102** that is permeable to oxygen, and an oxygen sorbent **103** situated within outer receptacle **101.** As provided herein, an inner collapsible blood container **102** is prepared from reinforced silicone membranes **600** or reinforced silicone membranes **700,** or combinations of the each. The present disclosure also provides for the manufacture of reinforced silicone membranes **600** or reinforced silicone membranes **700.**

The present disclosure also provides for, and includes, oxygen depletion devices **10** configured to be a blood collection and oxygen depletion device **10.** Oxygen depletion devices configured to collect and reduce blood oxygen differ from the oxygen depletion device **10** as described throughout this specification in that a blood collection and oxygen depletion device **10** further includes an anticoagulant to prevent coagulation of the whole blood during the collection process. In certain aspects, the anticoagulant solution of a blood collection and oxygen depletion device **10** is provided in the blood collection and oxygen depletion device **10.** Accordingly, included anticoagulant solutions are also oxygen depleted anticoagulant solutions. In the alternative, anticoagulant solutions may be included separately, either as oxygen depleted solutions or solutions having oxygen. A blood collection and oxygen depletion device **10** is intended to be used with whole blood collected from a donor.

As used herein, the outer receptacles are prepared from materials that are substantially impermeable to oxygen and optionally impermeable to carbon dioxide. In certain aspects, an outer receptacle **101** is prepared from flexible membrane materials. In other aspects, an outer receptacle **101** is prepared from a stiff, or inflexible membrane material.

The present disclosure provides for, and includes, an outer receptacle **101** substantially impermeable to oxygen. As used herein, an outer receptacle **101** that is substantially impermeable to oxygen is sufficiently impermeable to oxygen to allow no more than 10 cc of oxygen inside the receptacle over a period of 3 months, and more preferably no more than 5 cc of oxygen over 6 months. As used herein, the term substantially impermeable to oxygen (SIO) refers to materials and compositions that provide a barrier to the passage of oxygen from one side of the barrier to the other, sufficient to prevent significant increases in the partial pressure of oxygen. Outer receptacles **101** as used herein are described in detail in International Application Nos. PCT/US2016/02179 and PCT/US2016/029069.

The present disclosure provides for, and includes, the preparation of outer receptacles **101** and inner collapsible blood container **102** from a membrane or film. As used herein, membranes generally refer to materials used to prepare an inner collapsible blood container **102** and films are used to refer to materials used to prepare outer receptacle **101.** A membrane comprises one or more layers of materials in the form of a sheet that allows or prevents one or more substances to pass through from one side of the sheet to the other side of the sheet. As used herein, membranes may also be prepared as tubes suitable for connecting together components of oxygen depletion devices 10, blood collection kits, or connecting together elements of blood collection devices, additive solution bags, leukocyte reduction filters, and anaerobic storage bags. As used throughout, it is understood that a membrane of the present disclosure may be formed as a sheet or a tube depending on the application. Also as previously provided, membranes to prepare outer receptacles **101** are substantially impermeable to oxygen while an inner collapsible blood container **102** is permeable to oxygen.

As used herein, an inner collapsible blood container **102** is permeable to oxygen. In certain aspects, an inner collapsible blood container **102** is permeable to oxygen and carbon dioxide. In other aspects, an inner collapsible blood container **102** is permeable to oxygen and impermeable to carbon dioxide. Similarly, as used herein, reinforced silicone membranes **600** or reinforced silicone membranes **700** are permeable to oxygen. In other aspects, reinforced silicone membranes **600** or reinforced silicone membranes **700** are permeable to both oxygen and carbon dioxide. Unless specifically provided, the reinforced silicone membranes **600** or reinforced silicone membranes **700** are essentially impermeable to liquid water.

Membrane permeation flux, for a gas, is defined as the volume flowing through the membrane per unit area per unit time. The SI unit used is m³/m²·s. For gases and vapors, the volume is strongly dependent on pressure and temperature. Accordingly, permeation fluxes for gases are often given in terms of standard temperature and pressure (STP) which is defined as 0 °C and 1 atmosphere (1.0013 bar) (*e.g*., 273 °K and 760 torr). As noted above, the rate of passage depends on a driving force or difference between the two sides of the membrane, and this dependence is incorporated in the permeability coefficient, P, or simply the permeability.

Permeability (P) is defined as the permeability flux per unit of driving force per unit of membrane thickness. The SI unit for the permeability coefficient P is provided in Table 1. A common unit for gas separation, as in the present disclosure, is the Barrer and is also presented in Table 1. The term cm³ gas (STP)/cm²s refers to the volumetric trans-membrane flux of the diffusing species in terms of standard conditions of 0 °C and 1 atmosphere pressure, the term cm refers to the membrane thickness, and cm-Hg refers to the trans-membrane partial pressure driving force for the diffusing species. Permeability must be experimentally determined.

**Table 1: Permeability Units**

| | **Units of Permeability** |
|---|---|
| **"Volumetric" permeability** | $1 Barrer = \frac{{10}^{- 10} ⋅ {cm}^{3} gas \left(STP\right) ⋅ \left(cm membrane thickness\right)}{\left({cm}^{2} membrane area\right) ⋅ s ⋅ \left(cmHg pressure\right)}$ |
| **"Molar" permeability** | $\frac{mol}{m ⋅ Pa ⋅ s} \left(SI units\right) = \frac{\left({mol}_{i} permeating\right) ⋅ \left(m membrane thickness\right)}{\left({m}^{2} membrane area\right) ⋅ s ⋅ \left(Pa pressure\right)}$ |

Membranes suitable for the methods and devices according to the present disclosure include dense membranes, porous membranes, asymmetric membranes, and composite membranes. Dense membranes are membranes prepared from solid materials that do not have pores or voids. Materials permeate dense membranes by processes of solution and diffusion. Examples of dense membranes include silicone membranes (polydimethyl siloxane, or PDMS).

The present disclosure provides for, and includes, inner collapsible blood containers **102** prepared from membranes **113** that are characterized primarily by their permeability to oxygen. Unless indicated otherwise, a "substantially impermeable membrane" refers to membranes that are substantially impermeable to oxygen. However, in certain devices and methods, the membranes may be further characterized by the permeability or impermeability to carbon dioxide. For certain applications, the membrane material is substantially impermeable to oxygen and provides a barrier to the introduction of oxygen to the blood, blood component, or a blood collection kit comprised of multiple components. Such substantially impermeable membranes are generally used to prepare outer receptacles of the present disclosure. Suitable substantially impermeable membranes may also be used to prepare tubing for connective components of the devices and kits. Substantially impermeable membranes may comprise a monolayer or be laminated sheets or tubes having two or more layers.

The present disclosure also provides for, and includes, membranes **113** that are substantially permeable to oxygen. Membranes **113** that are substantially permeable to oxygen are used in the present disclosure for the preparation of inner collapsible blood containers **102.** In certain aspects, the membranes **113** that are permeable to oxygen are also biocompatible membranes, approved and suitable for extended contact with blood that is to be transfused into a patient. Like substantially impermeable membranes, substantially permeable membranes **113** may comprise a monolayer or may comprise a laminated structure having two or more layers. As provided herein, membranes **113** that are substantially permeable to oxygen are membranes **113** suitable for use in the preparation of reinforced silicone membranes **600** or reinforced silicone membranes **700.** Accordingly, except as modified to provided fabric reinforcement described below and throughout the specification as membranes **600,** or modified to provided silicone reinforcement described below and throughout the specification as membranes **700,** the permeability and other features of the membranes **113** (or **114)** are retained throughout.

In an aspect, oxygen permeable membranes **113** having a permeability to oxygen of greater than about 2.5 x 10⁻⁹ cm³ O₂ (STP)/((cm² s)*(cm Hg cm⁻¹) is used for the preparation of a collapsible blood container **102.** In another aspect, oxygen permeable membranes **113** having a permeability to oxygen greater than about 5.0 x 10⁻⁹ cm³ O₂ (STP)/((cm² s)*(cm Hg cm⁻¹) is used for the preparation of a collapsible blood container **102.** In yet another aspect, oxygen permeable membranes **113** have a permeability to oxygen of greater than about 1.0 x 10⁻⁸ cm³ O₂ (STP)/((cm² s)*(cm Hg cm⁻¹). In certain aspects, oxygen permeable membranes **113** suitable for use in the preparation of a collapsible blood container **102** are characterized by a Barrer value of greater than about 25. In other aspects, oxygen permeable membranes **113** suitable for use in the preparation of a collapsible blood container **102** are characterized by a Barrer value of greater than about 50. In certain other aspects, oxygen permeable membranes **113** suitable for use in the preparation of a collapsible blood container **102** are characterized by a Barrer value of greater than about 100.

In an aspect, a membrane **113** that is substantially permeable to oxygen can be dense membranes prepared from non-porous materials. Examples of suitable materials that are capable of high oxygen permeability rates include silicones, polyolefins, epoxies, and polyesters.

In aspects according to the present disclosure, a membrane **113** suitable for use in preparing an inner collapsible blood container **102** is a reinforced silicone membrane that is less than 100 µm thick and greater than 10 µm.

The present disclosure provides for, and includes, preparing membranes **113** that are substantially permeable to oxygen, not only by selecting the material, but also by selecting and controlling the thickness. As provided above, permeability is proportional to the thickness of the membrane. Accordingly, improved permeability may be achieved by decreasing the thickness of the membrane. In certain aspects, the minimum thickness is determined by its strength and resistance to puncture and tearing.

The present disclosure also provides for, and includes, membranes **113** that are substantially permeable to oxygen that are prepared using blow molding and injection molding techniques. Suitable materials for preparing inner collapsible blood containers **102** using blow molding and injection molding include silicone materials such as Bluestar 4350, 50 durometer, Silbione grade liquid silicone rubber and Shin-Etsu KEG-2000-40A/B Liquid Silicone. The silicone durometer choice is carefully chosen for collapsibility and permeability, followed by a well controlled wall thickness. Thinner materials will have a higher permeability and are less able to withstand puncturing, tearing, and dropping when formed into a container and filled with liquid. Methods to prepare blow molded and injection molded collapsible blood containers **102** are known in the art, for example, U.S. Patent 4,398,642 issued to Okudaira et al.; U.S. Patent 7,666,486 issued to Sato et al.; U.S. Patent 8,864,735 issued to Sano et al.; and U.S. Patent Application Publication No. 2012/0146266 by Oda et al*.* In an aspect, a blow molded collapsible blood container **102** can be prepared using LDPE used in the manufacture of collapsible water containers. As provided below, suitable blow molded or injection molded collapsible blood containers **102** have a permeability to oxygen of at least about 25 Barrer.

In an aspect according to the present disclosure, the collapsible blood container **102** can be manufactured from microporous membrane **113** by various sealing methods such as heat sealing, thermal staking, and adhesive bonding. In one aspect according to the present disclosure, a pair of PVDF microporous membranes are bonded together around the periphery with a section of PVC inlet tubing in place in the seam using an adhesive such as Loctite 4011 in conjunction with an adhesive primer such as Loctite 770. In another aspect according to the present disclosure, a collapsible blood container can be manufactured from a pair of microporous membranes by heat sealing the 3 or 4 edges of the pair of membranes together with a section of multilayer tubing sealed into the seam to provide for fluid connectivity.

The present disclosure provides for, and includes, a collapsible blood container **102** that is prepared from more than one type of membrane **113.** In an aspect, a collapsible blood container **102** comprises a first membrane **113** and a second membrane **114** suitably bonded to prepare a container. In another aspect, a collapsible blood container **102** comprises a membrane **113** combined with a second membrane **114** that has a permeability of less than about 30% of the permeability of first membrane **113.** In certain aspects, a second membrane **114** comprises a membrane that is relatively impermeable or insufficiently permeable to provide sufficient deoxygenation on its own, but can be combined with a suitable membrane **113.** In certain aspects, the second membrane **114** is relatively impermeable. In further aspects, the second membrane **114** comprises a molded membrane that incorporates ridges, baffles, or other structures to facilitate mixing. In an aspect, the second membrane **114** may comprise a rigid structure joined to an oxygen permeable membrane **113.** In aspects according to the present disclosure, the second membrane **114** is heat sealed to membrane **113.**

In certain aspects, the inner collapsible blood container **102** contains flow baffles located internal or external to the blood contact area that provide an increase in the turbulence inside the collapsible blood container **102** when agitated. In an aspect, baffles are located 1 to 2 inches from each other and comprise 10 to 45% of the inner collapsible blood container **102** area. In certain aspects, the flow baffles may comprise raised features **701** of a reinforced membrane **700** that are oriented internally in an inner collapsible blood container **102.** Accordingly, raised features **701,** suitably configured and placed on the internal surface of an inner collapsible blood container **102** provide an increase in the turbulence of the blood when agitated. When oriented on the internal surface of the inner collapsible blood container **102,** features **701** serve to both strengthen the membrane **113** and provide for improved mixing of the blood during deoxygenation.

In other aspects, the flow baffles may comprise fibers **601** of a reinforced membrane **600** that are oriented internally in an inner collapsible blood container **102.** Accordingly, raised fibers **601,** suitably configured and placed on the internal surface of an inner collapsible blood container **102** provide an increase in the turbulence of the blood when agitated. When oriented on the internal surface of the inner collapsible blood container **102** prepared from a reinforced silicone membrane **600,** fibers **601** serve to both strengthen the membrane **113** and provide for improved mixing of the blood during deoxygenation.

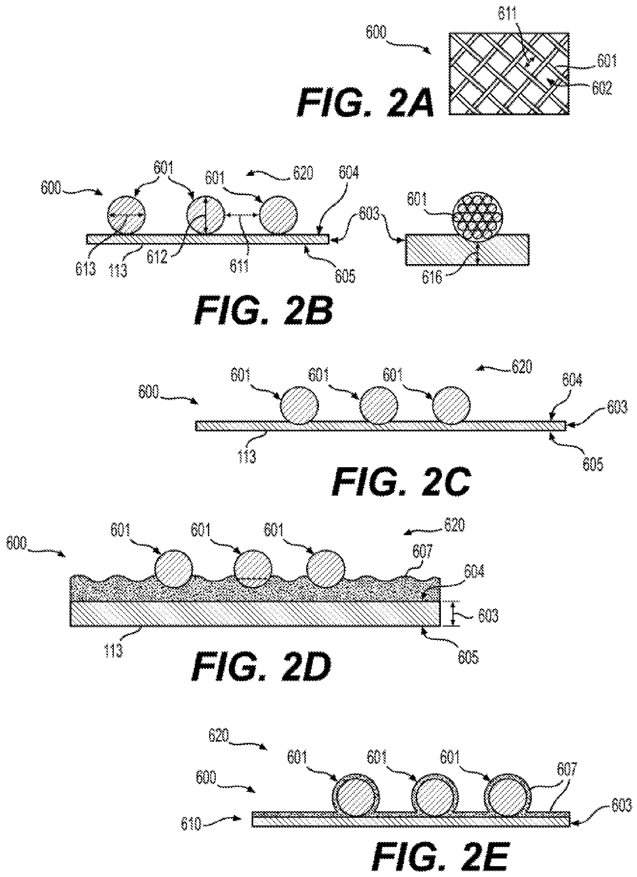

As provided herein, an inner collapsible blood container **102** may be prepared from silicone membrane **113** that has been reinforced with a fabric ("reinforced membrane **600"** or "membrane **600",** see Figure 2). Reinforced membranes **600** provide for the manufacture of inner collapsible blood container **102** that may help comply with ISO standard 3826-1:2013 that requires that plastic collapsible blood containers shall not show leakage when placed between two plates and subjected to an internal pressure of 50 kPa above atmospheric pressure for 10 minutes. When the thickness of a silicone membrane **113** is reduced below about 100 µm, the strength of the membrane is significantly compromised. Non-reinforced silicone membranes **113** are unsuitable for use in an oxygen depletion device **10** and the inner collapsible blood container **102** because once filled with blood (or liquid generally), they are subject to breakage when dropped. Such reduced thickness silicone membranes **113,** while suitable under controlled conditions are unsuitable for use under standard conditions present during blood collection and processing. At the same time, to achieve suitable rates of oxygen depletion in oxygen depletion devices 10 according the present disclosure, thinner thicknesses of silicone are desirable. As provided below, reinforced silicone membranes **113** that are 14 µm, 25 µm, and 50 µm thick when incorporated into an oxygen depletion device **10** provide suitable rates of oxygen transfer.

The present disclosure provides for, and includes, a collapsible blood container **102** that is substantially permeable to oxygen and is a membrane prepared from a reinforced membrane **600.** In aspects according the present disclosure, the collapsible blood container **102** can be prepared from a reinforced membrane **600** having a silicone thickness of between 5 µm and 100 µm. As used herein, a thickness of a reinforced membrane **600** refers to the thickness of the permeable membrane. As described below, the reinforcing fabric may be significantly thicker than the permeable membrane it reinforces. Also as used herein, the thickness of a blood container **102** refers to the thickness of the permeable membrane from which it constructed. In other aspects, the collapsible blood container **102** can have a thickness of between 5 µm and 75 µm. In other aspects, the collapsible blood container **102** can have a thickness of between 20 µm and 100 µm. In another aspect the collapsible blood container **102** is between 30 µm and 100 µm thick. In yet another aspect, the collapsible blood container **102** is between 50 µm and 100 µm thick. In a further aspect, the thickness of the collapsible blood container **102** can be between 20 µm and 75 µm. The present disclosure provides for, and includes, a collapsible blood container **102** that is 14 µm in thickness. In another aspect, the collapsible blood container **102** is 25 µm thick. In yet another aspect, the collapsible blood container **102** is 50 µm thick. In an additional aspect, the collapsible blood container **102** is 75 µm thick.

In aspects according the present disclosure, the collapsible blood container **102** can be prepared from a reinforced membrane **600** having a thickness of between 20 µm and 75 µm. In other aspects, the collapsible blood container **102** can have a thickness of between 20 µm and 50 µm. In other aspects, the collapsible blood container **102** can have a thickness of between 40 µm and 75 µm. In another aspect, the collapsible blood container **102** is between 40 µm and 50 µm thick. In yet another aspect, the collapsible blood container **102** can have a thickness of between 20 µm and 30 µm.

Suitable silicone membranes include commercially available membranes and membranes prepared from condensation or addition cured silicones. Non-limiting examples of silicone membranes are available from Wacker Silicones, such as the Silpuran^{®} brand of medical grade silicone sheet membranes (Wacker Silicones, Adrian, MI) and Polymer Sciences PS-1033 P-Derm^{®} silicone elastomer membrane (Polymer Sciences, Inc., Monticello, IN). In an aspect, the silicone membrane may be Polymer Sciences PS-1033 or Silpuran^{®} 6000 silicone. Silicone membranes can be prepared from various liquid silicone rubber (LSR) materials, which are available from a number of silicone suppliers, such as Wacker Silicones (Adrian, MI), Shin-Etsu Silicones of America (Akron, OH), NuSil Technology (Carpenteria, CA), and Blue Star Silicones (East Brunswick, NJ), to name a few.

Two part platinum cure liquid silicone rubber (e.g., condensation cured) and silicone dispersions are suitable for creating thin sheets for medical applications. Liquid silicone rubber (LSR); such as Wacker Silpuran 6000, Shin-Esu KEG2000, Dow Corning QP1, or NuSil MED-4901 is supplied with as a separate "A" component and "B" component that must be thoroughly mixed by the manufacturer's suggested method in order to initiate curing. Silicone dispersions, such as NuSil MED10-6640, are also supplied as an "A" component and "B" component that must be thoroughly mixed in order to initiate curing; however, these components are supplied in a suspension of a solvent, such as xylene. Xylene makes these silicones less viscous, which makes thin sheet fabrication easier. With all of these silicones, curing is accelerated with the application of heat.

One part Silicone RTV (room temperature vulcanizing), such as Wacker Silpuran 4200, can also be used to create sheets. One part silicones of the RTV type cure at ambient temperature using the moisture in air, generally have a long cure time and may not be preferred for large scale manufacturing using the methods described below. In certain aspects, one part silicone RTV can be used to prepare frames **120** as described below suitable for joining a reinforced membrane **600** to prepare inner collapsible blood containers **102.** In other aspects, one part silicone RTV can be used to prepare frames **120** as described below suitable for joining a reinforced membrane **700** to prepare inner collapsible blood containers **102.** Curing times may also be increased by introducing moist air that can accelerate the curing process.

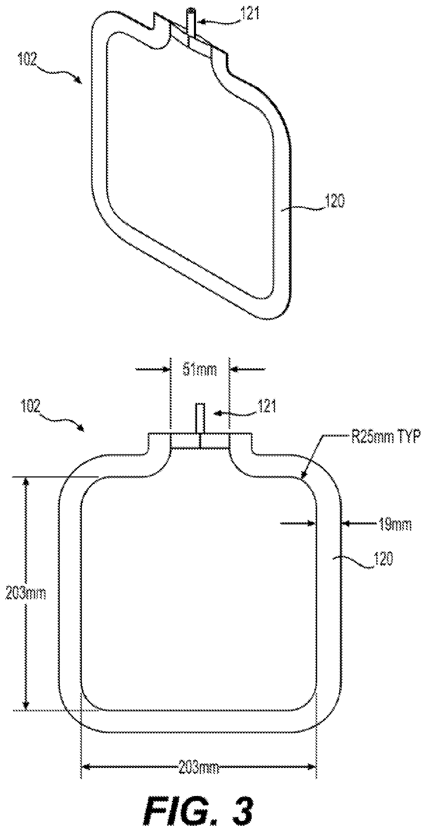

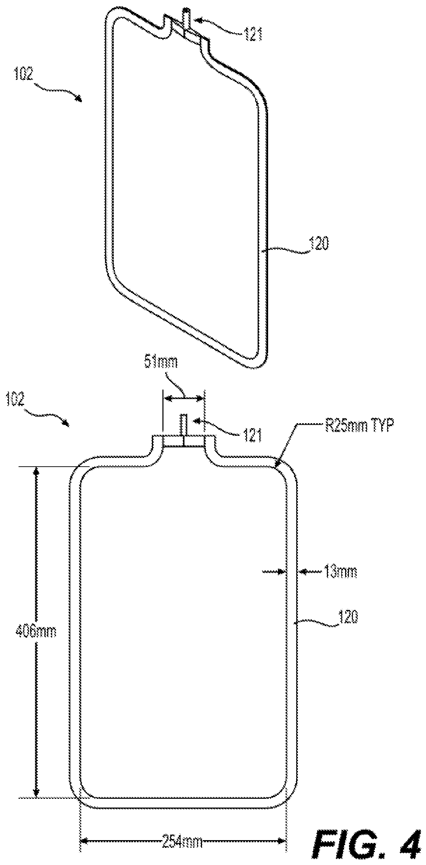

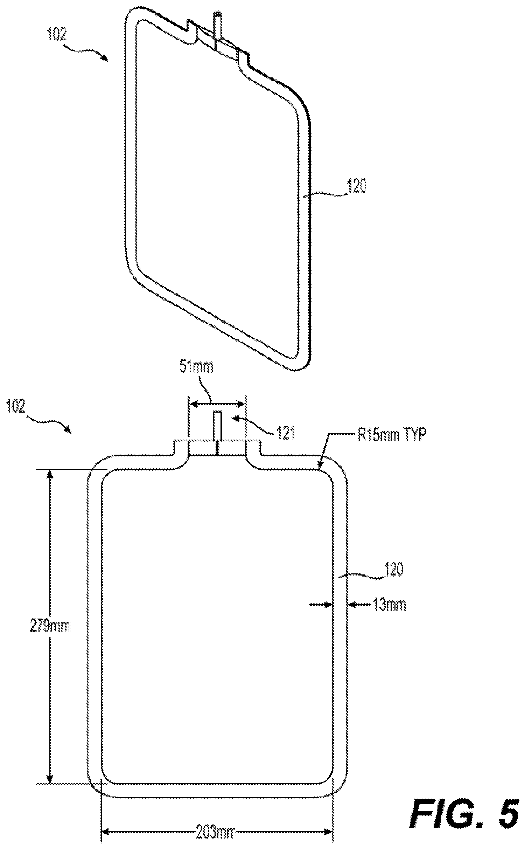

In aspects according to the present disclosure, two part platinum cure high consistency rubber (HCR) silicone, such as NuSil MED-4050 or SIL2-5070, may be used to create a frame **120** that joins two reinforced silicone sheets (reinforced membranes **600** or **700)** to create an inner collapsible blood container **102.** (See Figures 3 to 5, illustrated as item 120). An advantage of HCR is a very high viscosity, improving to manipulability and handling during fabrication. HCR is supplied by the manufacturer in two components that must be thoroughly mixed by the manufacturer's suggested method in order to initiate curing. Heat can be applied to the mixed resin in order to accelerate curing once a container is fabricated. Examples of the preparation of reinforced membrane **600** using heat regimens to partially cure the silicone are provided below. The frame **120** becomes and integral component of the inner collapsible container **102.**

In accordance with the invention, a reinforced membrane **600** comprises a thin silicone layer having high oxygen permeability and a fabric reinforcing layer. Reinforced membranes **600** are suitable for the preparation of inner collapsible containers **102** for use in oxygen depletion devices **10.** The reinforced silicone membranes are further characterized as having a relatively smooth surface for contact with the blood or blood component for depletion. In certain aspects, additional features may be introduced into the blood contacting surface of a reinforced membrane **600** to provide for additional mixing (*i.e.,* features in addition to fabric **601,** that when placed in contact with blood and aid in mixing).

Silicone is generally resistant to attachment to non-silicone materials. Accordingly, the present disclosure provides for, and includes, methods for joining the reinforcing fabric material to the silicone. The resulting reinforced membranes **600** are then used for the production of inner collapsible containers **102.** As described below, the process of preparing reinforced membranes **600** and incorporating them into inner collapsible containers **102** can be automated for large scale industrial production, either in batch mode or as continuous production line.

Referring to Figures 2A to 2E, the various processes for the preparation of reinforced membranes **600** result in membranes **600** having differing overall geometries. As used throughout, membranes **600** are reinforced membranes **600.** In embodiments outside the scope of the claims, membranes **600** may also include reinforced membranes prepared from other, non-silicone materials. Figure 2A shows a top view of the reinforcing fabric **600,** wherein the fabric has discrete fibers **601** and open areas **602** between the fibers. The fibers **601** are spaced at least about 0.1 mm apart to provide gas permeability of the open areas **602,** but no more than about 4 mm apart to provide the reinforcement strength needed.

The present disclosure provides for, and includes a membrane **600** as illustrated in Figure 2B. Now referring to Figure 2B, which is a cross-section view of a membrane **600** showing the discrete fibers **601** of a reinforcing fabric **620** and a silicone membrane **603** (*e.g.,* a membrane **113),** having an outer surface **604** and an inner surface **605,** wherein the reinforcing fabric **620** (comprising fibers **601)** is placed on the outer surface **604** of the silicone membrane **603.** In an aspect of the invention, the silicone membrane **603** is partially cured and contains a suitable solvent, such as xylene, and has a thickness of about 30-75 µm before complete curing and removal of solvent. In another aspect of the invention, the silicone membrane **603** is fully cured and contains no solvent.

The present disclosure provides for, and includes a membrane **600** as illustrated in Figure 2C. Figure 2C is a cross-section view of a membrane **600,** having a reinforcing fabric **620** and a silicone membrane **603,** showing discrete fibers **601** of the reinforcing fabric **620.** The membrane **600** of Figure 2C is representative of Figure 2B having a partially cured silicone membrane **603** after pressing the reinforcing fabric **620** into the outer surface **604** of the partially cured silicone membrane **603** and heating the structure to fully cure the silicone membrane **603** and remove the solvent. The discrete fibers **601** are embedded in and attached to the cured silicone membrane **603,** but do not protrude through the inner surface **605.** In an aspect of the invention, the membrane **600** of Figure 2C provides a gas permeable material having sufficient strength for routine handling when used in an inner collapsible container **102.**

The present disclosure provides for, and includes a membrane **600** as illustrated in Figure 2D that is a cross section view of a membrane **600,** having a reinforcing fabric **620** and a silicone membrane **603,** showing discrete fibers **601** of the reinforcing fabric, and further having a bonding layer **607.** The bonding layer **607** is comprised of a silicone LSR, and optionally a suitable solvent such as xylene. In aspects according to the present disclosure, the uncured bonding layer **607** is dispensed onto a fully cured silicone membrane **603,** such as by spraying or knife coating, to yield a thin layer having a thickness of about 10-50 µm before placing the reinforcing fabric **620** onto the uncured bonding layer **607.** The membrane **600** is then heated to about 115-121°C, or according to manufacturer's instructions, to completely cure the bonding layer **607.** The fully cured membrane **600** is suitable for use in an inner collapsible container **102.**

Figure 2E is a cross section view of a fabric reinforced silicone membrane **600,** having a reinforcing fabric **620** and a silicone membrane **603,** showing discrete fibers **601** of the reinforcing fabric, and further having a bonding layer **607.** Silicone layer **610** is comprised of silicone membrane **603** and bonding layer **607.**

The reinforcing fabric **620** is first placed onto a fully cured silicone membrane **603,** and a bonding layer **607** is comprised of an uncured silicone LSR, and optionally a suitable solvent such as xylene is dispensed, such as by spraying, to yield a thin coating having a thickness of about 10-50 i.tm onto the reinforcing fabric **620** and the fully cured silicone membrane **603.** The resulting membrane **600** is then heated to about 115-121°C to completely cure the bonding layer **607.**

In another aspect according to the present disclosure, a reinforcing fabric **620** is dipped in an uncured silicone LSR, and optionally a suitable solvent such as xylene, before placing the reinforcing fabric **620** onto a fully cured silicone membrane **603,** followed by curing with heat to yield the structure shown in Figure 2E.

The present disclosure provides for, and includes, reinforced membranes **600** that are reinforced with a fabric. As used herein, a "fabric" refers to a woven or non-woven fabric or mesh. Also provided for and included in the present disclosure are silicone membranes **113** having fabrics that are configured as a mesh. As used herein, a "mesh" refers to a network of spaces in a net or network comprising a network of cords or threads. In some aspects, a mesh may be a woven cloth or fabric. In other aspects, a mesh may be a nonwoven cloth or fabric. As used herein, fabrics are distinguishable from the meshes used as a spacer **110.**

Attaching a reinforcing fabric to a silicone membrane can be achieved by various methods as previously described, including casting, coating, and spot bonding. As noted above, adhesion of silicone to some materials can be low resulting in resistance to attachment of the reinforcing fabric. As provided above, fabrics can be bonded by embedding, partially, or completely the fabric in silicone during manufacture.

The present disclosure provides for and includes methods to enhance the bonding or adhesion of the fabric to the silicone membrane **113** to prepare membranes **600.** In an aspect, the adhesion of the fabric to the silicone can be enhanced by plasma treatment of either or both of the materials to be bonded. Plasma treatment for adhesion promotion is well known by one skilled in the art, and suitable processes include vacuum plasma, corona discharge, and atmospheric pressure plasma processing. The plasma treatment of the material before bonding provides for the creation of reactive groups on relatively inert surfaces of materials such as silicones and polyolefins, as well as provides for the removal of surface contaminants from these surfaces. Suitable equipment to treat the materials with atmospheric pressure plasma include the Openair^{®} plasma systems (Plasmatreat USA, Elgin, IL) and the ULD plasma curtain from AcXys Technologies (Le Vinoux, France).

Fabrics for preparing reinforced membranes **600** according to the present disclosure may be prepared from polymers, carbon fibers, fiberglass, natural fibers, and other materials that can be prepared as a mesh. Fabrics may be woven meshes prepared from monofilament synthetic or natural fibers or yarns. In other aspects, woven fabrics may be prepared from multifilament synthetic fibers or yarns.

In an aspect the fabric may be nylon, polybutylene terephthalate (PBT), polyester, polyethylene, polypropylene, polytetrafluoroethylene (PTFE), polypropylene/polyethylene (PP/PE) blends or synthetic yarns or fibers. In an aspect, the material for the preparation of fabrics for preparing reinforced membranes **600** is a polyester fabric. In an aspect, the material for the preparation of fabrics for preparing reinforced membranes **600** is a nylon fabric. In an aspect, the material for the preparation of fabrics for preparing reinforced membranes **600** is a polyethylene fabric. Exemplary fabrics suitable for the preparation of a reinforced membrane **600** include polyester fabrics from Textile Development Associates, Surgical Mesh Division. Suitable fabrics include, but are not limited to catalog numbers PETKM2002, PETKM2004, PETKM2005, PETKM2006, PETKM2007, PETKM3002, PETKM3003, PETKM7002, PETKM14002, and PETKM22002. Additional exemplary polyester fabrics are catalog numbers P20D, P118, P201, PR150, D117, D1171, D1400, D2000 available from Mohawk Fabrics, Amsterdam, NY 12010.

The fabric can be woven or non-woven, and the fiber size and spacing can be varied to provide a suitable open area for the desired gas permeability while providing enhanced strength to the silicone membrane. Suitable open areas range from about 0.1 to about 3.0 square mm, with fabric fiber size ranging from about 11 to 163 grams per square meter (GSM).

In aspects according to the present disclosure, a fabric suitable for preparing a reinforced membrane **600** may be prepared from natural fibers including cotton and wool. In some aspects, the natural fiber is seed fiber, a leaf fiber, a bast fiber, a skin fiber, a fruit fiber, or a stalk fiber. In other aspects, the natural fiber is hemp, sisal, jute, kenaf, or bamboo. In an aspect, the fabric may be prepared from silk.

In aspects according to the present disclosure, a fabric may be an extruded fabric (also called "extruded netting"). In an aspect, an extruded fabric may be a bi-planar extruded fabric. In another aspect, the extruded fabric may be a mono-planar fabric. Extruded fabric may comprise a netting having a variety of apertures (hole sizes), weights, and thicknesses. Extruded fabrics may be prepared from polypropylene (PP), polyethylene (PE), high density polyethylene (HDPE), medium-density polyethylene (MDPE), low-density polyethylene (LDPE), polypropylene/polyethylene (PP/PE) blends, cross-linked polyethylene (PEX), ultrahigh molecular weight polyethylene (UHMWPE).

The reinforcing fabric used to strengthen the silicone membrane can be made from various natural and synthetic materials and fibers, including cotton, silk, wool, polyesters including Dacron^{®}, polyolefins including polyethylene and polypropylene, nylons, polyurethanes, acrylics, cellulose, cellulose acetate, Rayon, polyvinylchloride (PVC) and aramids including Kevlar^{®} Nomex^{®} and Technora^{®}. The fabric can be woven or non-woven, and the fiber size and spacing can be varied to provide a suitable open area for the desired gas permeability while providing enhanced strength to the silicone membrane.

Woven fabrics of the present disclosure may be described by the thread count and have a thread diameter. Woven fabrics comprise warp threads that run lengthwise , and weft or filling threads that run across the width of a fabric at right angles to the warp thread. In woven fabrics comprising monofilaments, equal diameter threads and equal thread counts are present in both the warp and weft directions and square mesh openings (or holes). Monofilament woven fabrics may have different numbers of thread counts in the warp and weft direction resulting in rectangular fabric openings. Woven fabrics are available in a wide variety of thread counts.

In aspects according to the present disclosure, the fabric **620** is between about 50 micrometers (µm) and about 1.5 mm in total thickness. In certain aspects, the maximum thickness of the fabric 750 µm to about 1.0 mm. In an aspect, the thickness of the fabric **620** is between 150 and 300 µm. In an aspect, the thickness of the fabric **620** is between 100 and 450 µm. In an aspect, the thickness of the fabric **620** is between 50 µm and 300 µm. In another aspect, the thickness of the fabric **620** is between 50 µm and 200 µm. In an aspect, the thickness of the fabric **620** is between 200 µm and 300 µm. In an aspect, the thickness of the fabric **620** is about 150 µm. In an aspect, the thickness of the fabric **620** is about 200 µm. In an aspect, the thickness of the fabric **620** is about 250 µm. In an aspect, the thickness of the fabric **620** is about 300 µm.

In the course of developing reinforced membranes **600** of the present disclosure, it is observed that fabrics **620** require a mesh having an open area of more than 75% do not provide a sufficient reinforcement of the silicone to prevent rupture, tearing or puncture, for example when tested in a drop test described in Example 5. Accordingly, the present disclosure provides for and includes, fabrics having a mesh with an open area of between 20% and 60% and a maximal thickness of up to 750 µm. Also included are fabrics having a mesh with an open area of about 55%. In an aspect the mesh opening is about 200 microns and the thread thickness is about 152 microns.

In aspects according to the present disclosure, a fabric **620** may be prepared having a regular, repeating pattern of spaces in the net or network. In other aspects, a fabric **620** of the present disclosure may have an irregular or non-repeating pattern of spaces. In yet another aspect, the fabric **620** may be a random array of open spaces. In another aspect, the fabric **620** may have a honeycomb appearance. In aspects according to the present disclosure, the open spaces within the fabric **620** are round, triangular, square, polygonal, polyhedron, ellipsoid, or spherical.

According to the present disclosure, the fabric **620** comprises a fabric **620** having a percentage of open area of between 40% and 60%. In another aspect, the fabric **620** may have an open area of between 20% and 30%. In an aspect, the fabric **620** may have an open area of between 30% and 40%. In a further aspect, the fabric **620** may have an open area of between 40% and 50%. In yet another aspect, the fabric **620** may have an open area of between 50% and 60%. In certain aspects, the percentage of open area of the fabric **620** may be between 36% and 38%. In an aspect, the percentage of open area is about 37%.

In other aspects, the fabric **620** has a thickness of between 150 µm and 300 µm and has an open area of a fabric **620** between 50% and 70%. In another aspect, the fabric **620** has a thickness of between 150 µm and 300 µm and has an open area of a fabric **620** between 55% and 60%.

Woven monofilament fabrics suitable for the preparation of reinforced silicone membranes **113** of the present disclosure comprise fabric **620** having nominal hole sizes (*e.g.,* mesh openings) ranging from 0.1 to 3 mm².

In aspects according to the present disclosure, suitable fabrics **620** include woven or non-woven fabrics having a pore size of between 0.1 square millimeters (mm²) to about 3.0 mm². As provided herein, fabrics **620** of the present disclosure may have a strand thickness of between 0.15 mm to 0.3 mm. To ensure proper permeability, the fabrics **620** of the present disclosure have an open area of between 50% to 70%. In an aspect, the fabric **620** for reinforcing the silicone membrane is a fabric **620** that has an opening of 1 mm² and a strand thickness of 0.2 mm, and open area of about 55%. In an aspect, the fabric **620** has a strand thickness of 0.0254 millimeters (1 mil). In another aspect, the fabric **620** has a strand thickness of 0.0127 mm (0.5 mil). Suitable fabrics **620** provide for membranes **600** that when incorporated into an inner collapsible blood container **102** that can withstand drop testing from a height of about 6 feet.

In an aspect according to the present disclosure, a collapsible blood container **102** can be manufactured from silicone by various molding methods such as compression molding, injection molding, and insert molding, and also adhesive bonding of silicone sheets using silicone adhesives. In one aspect according to the present disclosure, a pair of silicone sheets are bonded together around the periphery with a section of silicone inlet tubing in place in the seam using silicone adhesive. In another aspect according to the present disclosure, a silicone liquid rubber is injection molded over a form to create a three-sided shape, which is then further bonded to closure on the remaining fourth side around a silicone inlet tube using a silicone adhesive. In another aspect according to the present disclosure, a silicone liquid rubber is injection molded over a form to create a three-sided shape, which is then insert molded onto a closure shape on the remaining fourth side that incorporates an inlet tubing into the closure shape.

The present disclosure provides for, and includes, a collapsible blood container **102** having resistance to tearing. As used herein, "tear resistance" or "tear strength" is measured in kN/m. In aspects according the present disclosure, the collapsible blood container **102** should be prepared from oxygen permeable materials that are also resistant to tearing. Measures of tear resistance are known in the art, for example, ASTM D-412, which can also be used to measure tensile strength, modulus, and elongations. In certain aspects, collapsible blood container **102** should be prepared from oxygen permeable materials that are resistant to the formation of a tear (*e.g*., tear initiation). Methods of measuring tear initiation and tear propagation are known in the art, for example ASTM D-624. Other methods include measuring the tensile strength and the elongation at break according to DIN 53 504-S1.

In an aspect according to the present disclosure, a collapsible blood container **102** should be prepared from oxygen permeable materials having a tensile strength of at least 2.4 N/mm².

The present disclosure provides for, and includes, sorbents capable of binding to and removing oxygen from an environment. Unless provided otherwise, the term "sorbent" refers to oxygen sorbents and scavengers. As used herein, "oxygen scavenger" or "oxygen sorbent" is a material that binds irreversibly to or combines with O₂ under the conditions of use. The term "oxygen sorbent" may be used interchangeably herein with "oxygen scavenger." In certain aspects according the present disclosure, a material may bind to or combines with oxygen irreversibly. In other aspects, oxygen may bind to a sorbent material and have a very slow rate of release, k_{off}. In an aspect, the oxygen may chemically react with some component of the material and be converted into another compound. Any material where the off-rate of bound oxygen is much less than the residence time of the blood can serve as an oxygen scavenger. Suitable sorbents as used herein are described in detail in International Application Nos. PCT/US2016/02179 and PCT/US2016/029069.

As used herein, "carbon dioxide scavenger" is a material that binds to or combines with carbon dioxide under the conditions of use. The term "carbon dioxide sorbent" may be used interchangeably herein with "carbon dioxide scavenger." In certain aspects, carbon dioxide sorbents may be non-reactive, or minimally reactive with oxygen. In other embodiments, oxygen sorbents may exhibit a secondary functionality of carbon dioxide scavenging. Carbon dioxide scavengers include metal oxides and metal hydroxides. Metal oxides react with water to produce metal hydroxides. The metal hydroxide reacts with carbon dioxide to form water and a metal carbonate. In certain aspects according the present disclosure, a material may bind to or combine with CO₂ irreversibly. In aspects according to the present disclosure, a material may bind CO₂ with higher affinity than hemoglobin. In other aspects, a sorbent material may bind CO₂ with high affinity such that the carbonic acid present in the blood or RBC cytoplasm is released and absorbed by the sorbent. In other aspects, CO₂ binds to a sorbent material and has a very slow rate of release, k_{off}. In an aspect, the carbon dioxide can chemically react with some component of the material and be converted into another compound. Suitable carbon dioxide scavengers as used herein are described in detail in U.S. Provisional Application Nos. 62/131,130 and 62/151,957.

The users of the collapsible container require convenient filling and removal of the contents, and must be able to empty the contents within 2 minutes per the ISO 3826 standard for blood containers. The outer receptacle can reduce the filling time by constraining the collapsible container and preventing it from expanding. Thus, in some embodiments, the blood storage device is further comprised of an expansion feature to allow for unrestricted filling of the collapsible container. In some embodiments the expansion feature is comprised of a gusseted fold along one or more edges of the outer receptacle. Typically, a fold of about ¼ inch is adequate to provide for expansion of the inner container, and the pleats of the fold are sealed into the seams at the ends. In some embodiments, the expansion feature is comprised of a third panel of barrier film sealed along the bottom of the outer receptacle, providing for a three-dimensional bag.

During the development of the oxygen depletion device **10,** it was discovered that the size, shape, and number of chambers of an inner collapsible blood container **102** needed to be controlled in order to obtain suitable depletion kinetics. More particularly, even using highly permeable materials, using standard blood bag configurations proved inadequate and had significantly slower reaction kinetics. Not to be limited by theory, it is hypothesized that deoxygenation is a multistep process including release of dissolved oxygen from hemoglobin, diffusion of the dissolved oxygen within the red blood cell cytoplasm, and diffusion of the dissolved oxygen through the red blood cell membrane. Also not to be limited by theory, it is hypothesized that the high concentration of hemoglobin, having very high affinity for oxygen, greatly decreases the diffusion rate of the dissolved oxygen within the cytoplasm. Similarly, the diffusion of dissolved oxygen once it passes through the plasma membrane to the plasma is further limited by absorption and binding to other red cells. Again, not to be limited by theory, it is hypothesized that an additional diffusion barrier for the dissolved oxygen occurs at the gas permeable membrane where it not only needs to pass through the membrane, but also changes state from the dissolved phase to the gaseous phase. Subsequent diffusion and adsorption by the sorbent occurs in a gaseous state and is maximized by incorporating and maintaining a headspace within the outer receptacle **101.** Accordingly, it is believed that the diffusion of the gaseous oxygen is maximized by maintaining the concentration gradient within the headspace from the surface of the inner collapsible blood container **102** to the oxygen sorbent **103.** Also not to be limited by theory, it is thought that by selecting sorbents that have high absorption kinetics, high binding capacity, and combinations of both, a suitable diffusion gradient for the gaseous oxygen is maintained to drive the rapid kinetics of oxygen depletion in oxygen depletion device **10.**

The present disclosure provides for, and includes, an oxygen depletion device **10** for depleting oxygen from blood that comprises an inner collapsible blood container **102** having a surface to volume ratio of between 0.05 centimeters²/milliliter (cm²/ml) and 5.0 cm²/ml enclosed within an outer receptacle **101.** In certain aspects, an oxygen depletion device **10** for depleting oxygen from blood comprises an inner collapsible blood container **102** having a surface to volume ratio of between 0.08 cm²/ml and 4.0 cm²/ml enclosed within an outer receptacle **101** when filled with blood for oxygen depletion. In some aspects, an oxygen depletion device **10** for depleting oxygen from blood comprises an inner collapsible blood container **102** having a surface to volume ratio of between 0.09 cm²/ml and 3.8 cm²/ml enclosed within an outer receptacle **101** when filled with blood for oxygen depletion.

As used herein, surface to volume ratios are defined with respect to a standard unit of whole blood, about 1 pint or 450-500 ml. As is evident to a person of skill in the art, collection of less than a unit of blood results in an even lower surface to volume ratio and the oxygen depletion device **10** is suitable for collecting a fraction of a unit of blood without modification. For the collection of more than a unit of blood, the size of the collapsible blood container **102** would need to be adjusted to provide for the desirable rapid kinetics of blood depletion. Modifications of the sort necessary to adapt an oxygen depletion device **10** for the collection of more than a unit of blood is within the level of ordinary skill in the art.

The present disclosure further includes and provides for oxygen depletion device **10** for the collection and depletion of packed red blood cells. A full unit of packed red blood cells in an additive solution comprises about 280 ± 60 ml.

In an aspect according to the present disclosure, the surface to volume ratio of a collapsible blood container **102** is at least 0.9 centimeters²/milliliter (cm²/ml) when filled with blood for oxygen depletion. Not to be limited by theory, it is believed that by increasing the surface to volume ratio, the diffusion limitations imposed by blood itself, particularly by the red blood cells and hemoglobin, can be overcome by decreasing the diffusion distance of the dissolved oxygen within the inner collapsible blood container **102.** In an aspect, the surface to volume ratio of a blood container **102** is at least 1.0 cm²/ml when filled with blood for oxygen depletion. In another aspect, the surface to volume ratio of a collapsible blood container **102** is at least 1.5 cm²/ml when filled with blood for oxygen depletion. In a further aspect, the surface to volume ratio of a collapsible blood container **102** is at least 2.0 cm²/ml when filled with blood for oxygen depletion. In some aspects, the surface to volume ratio of a collapsible blood container **102** is at least 3.0 cm²/ml when filled with blood for oxygen depletion. In yet other aspect, the surface to volume ratio of a collapsible blood container **102** is at least 4.0 cm²/ml when filled with blood for oxygen depletion.

The present disclosure also includes and provides for increasing the kinetics of deoxygenation of blood by modifying the dimensions of the inner collapsible blood container **102.** Not to be limited by theory, the average diffusion distance of a red blood cell in blood minimized as the height is decreased leading to increased deoxygenation kinetics. In certain aspects according the present disclosure, the collapsible blood container **102** is 12.5 cm by 17.5 cm by 0.002 cm before filling with blood, and about 2.0 cm in height after filling with blood. In other aspects according the present disclosure, the collapsible blood container **102** is 17.5 cm by 28.0 cm by 0.04 cm before filling with blood, and about 2.0 cm in height after filling with blood. In other aspects according the present disclosure, the collapsible blood container **102** is 25.0 cm by 60.0 cm by 0.04 cm before filling with blood, and about 0.3 cm in height after filling with blood.

In certain aspects, the height of a collapsible blood container **102** is no greater than 0.002 cm. In an aspect the height of a collapsible blood container **102** is no greater than 0.04 cm. In certain aspects, the height of a collapsible blood container **102** is between 0.002 and 0.04 cm. When filled with blood, the height of a collapsible blood container **102** is no greater than 0.3 cm. In an aspect the height of a collapsible blood container **102** when filled with blood is no greater than 1.5 cm. In certain aspects, the height of a collapsible blood container **102** when filled with blood is between 0.3 cm and 2.5 cm.

The present disclosure also includes and provides for an oxygen depletion device **10** having dimensions suitable for incorporation of existing blood collection protocols using existing equipment. Design of an oxygen depletion device **10** with recognition to existing technologies reduces capital costs in centralized processing centers and further provides for increased consistency and reliability. As used herein, the dimensions of an oxygen depletion device **10** is primarily limited to the length and width of the outer receptacle **101** where the height of the bag is determined by the requirements of the collapsible blood container **102** to contain about a pint or 450 to 500 ml. of blood, which is equivalent to a "unit of blood". The height of an oxygen depletion device **10** is further constrained by the presence of one or more sorbent packets and devices included to maintain an appropriate headspace. In view of these considerations, it become apparent that constraints on the dimension of the outer receptacle **101** of an oxygen depletion device **10** necessarily limits the dimensions of a collapsible blood container **102.** Accordingly, a collapsible blood container **102** may be divided into one or more chambers in fluid communication with each other.

In aspects according to the present disclosure, an oxygen depletion device **10** is designed to be incorporated into existing blood agitation equipment. In certain aspects, an oxygen depletion device **10** is dimensioned to efficiently utilize the space available in agitator and mixing tables. In an aspect, an oxygen depletion device **10** is dimensioned to maximally utilize the area available in a platelet agitator, for example a Helmer Labs Platelet Agitator, Model PF96. Suitable dimensions of an oxygen depletion device **10** include those that allow for 1, 2, 4, 6, 8, 10 or more bags to be placed on a flat agitator or mixer surface.

In an aspect, the area of an oxygen depletion device **10** lying flat is between 150 and 250 cm². In another aspect an oxygen depletion device **10** lying flat is between 450 and 550 cm². In another aspect an oxygen depletion device **10** lying flat is between 1400 and 1500 cm². . In another aspect an oxygen depletion device **10** lying flat is between 150 and 1500 cm².

As is evident, an oxygen depletion device **10** having a defined size necessarily constrains the dimensions of a collapsible blood container **102** according to the present disclosure. In certain aspects, a collapsible blood container **102** is further limited by a specified surface to volume ratio. In accordance with these limitations, the present disclosure provides for, and includes, a collapsible blood container **102** having two or more chambers in fluid communication with each other.

The oxygen depletion container device can be constructed in such a manner that allows for the blood volume to area of bag to be optimized against the overall size of the oxygen depletion container device, while exposing more of the blood volume to the material with oxygen permeability in the utilized space. The blood volume can be contained in a collapsible blood container **102** having two or more chambers that allow for their specific arrangement within the outer receptacle **101.** In certain aspects, the oxygen depletion device **10** height, when placed onto a surface, does not occupy impractical space in the intended mixing apparatus. The chambers can be arranged side to side, stacked on top of one another, partially stacked onto each other, staggered in a row, or saddled on top of each other onto one or more stacking heights. Sorbent **103** can be positioned over or between chambers as needed. Chambers may be filled and drained individually or in unison when such chambers are connected via tubing or fluid conduits that allow for easy filling and draining. It would be understood that the arrangement and interconnection of collapsible blood containers **102** having two or more chambers can be performed by a person of skill in the art.

In certain aspects, a collapsible blood container **102** comprises two or more chambers. In an aspect, a collapsible blood container **102** can have two chambers placed side by side or end to end depending on the dimensions. In another aspect, a collapsible blood container **102** can have three chambers placed side by side or end to end depending on the dimensions. In yet another aspect, a collapsible blood container **102** can have three chambers placed side by side or end to end depending on the dimensions. A person of ordinary skill could prepare additional configurations of a collapsible blood container **102** having multiple chambers placed in adjacent positions and orientations to maximize the utilization of space.

In other aspects provided for and included in the present disclosure, a collapsible blood container **102** may comprise two or more chambers that are stacked. When in a stacked configuration, to maintain optimal gas diffusion rates, spacers or meshes are included to ensure the separation of adjacent chambers. In certain aspects, the space between a stacked chamber further includes one or more sorbent sachets in order to maintain optimal gas diffusion rates. In certain aspects, two chambers may be stacked. In another aspect, three chambers may be stacked. In yet another aspect, four chambers may be stacked.

The present disclosure provides for, and includes, a collapsible blood container **102** comprising a combination of stacked and adjacent chambers. As provided herein, the number and stacking of chambers of a collapsible blood container **102** further comprises a surface to volume ratio of the combined chambers of at least 0.4 cm²/ml. Additional variations consistent with the present disclosure can be prepared by one of ordinary skill in the art.

The present invention provides an oxygen depletion device **10** for depleting oxygen from blood comprising an outer receptacle **101** substantially impermeable to oxygen, inner collapsible blood container **102** that is permeable to oxygen and an oxygen sorbent situated within said outer receptacle. The collapsible blood container **102** may further comprise one or more mixing structures that increase mixing of the blood during oxygen depletion. In certain aspects, the mixing structures are incorporated into the structure of the collapsible blood container **102.** In other aspects, the mixing structures are added to the inside of, but not physically joined to the collapsible blood container **102.** In yet other aspects, a mixing structure is a structure outside of the collapsible blood container **102** that restricts or modifies the shape of the container **102** to decrease or disrupt laminar flow. Mixing structures according to the present disclosure are designed to increase blood movement in the collapsible blood container **102,** increase turbulent flow within the collapsible blood container **102,** or combinations of both. Importantly, mixing structures and mixing should not significantly increase lysis, or damage to, the red blood cells.

In aspects according to the present disclosure, a mixing structure is included in the structure of membrane **113.** In certain aspects, a mixing structure in membrane **113** comprises ridges, bumps, or protrusions on the inside of the collapsible blood container **102** and are in contact with the blood. As provided herein, such mixing structures can include the fabric reinforced surface of a reinforced membrane **600** or the features **701** of a reinforced membrane **700** when the surfaces are incorporated on the inside surface of a collapsible blood container **102.** In certain aspects, the reinforcing features **701** may be present on both sides of a silicone membrane **113** of a reinforced membrane **700** wherein the features **701** provide both reinforcing functions and mixing functions. In an aspect, a mixing structure in membrane **113** comprises one or more ridges. In an aspect, the one or more ridges extend across the full width or length of the inner surface of collapsible blood container **102.** In other aspects, the ridges alternate and may be staggered. In certain aspects, the mixing structure in membrane **113** comprises bumps or other protrusions designed to disrupt laminar flow and induce turbulence. Similarly, in certain aspects, the mixing structure in membrane **113** comprises depressions designed to disrupt laminar flow and induce turbulence. In certain aspects, the mixing structures are baffles incorporated into membrane **113.** Baffles are flow directing vanes or panels. In some aspects, a mixing structure comprising one or more baffles may be incorporated into a second membrane **114.**

In certain aspects, a mixing structure is contained within the collapsible blood container **102.** In an aspect, a mixing structure within the collapsible blood container **102** comprises one or more beads or balls that aid in mixing when the collapsible blood container **102** is agitated. In another aspect, a mixing structure within the collapsible blood container **102** comprises one or more strings or elongated structures that aid in mixing when the collapsible blood container **102** is agitated. In yet another aspect, a mixing structure within the collapsible blood container **102** comprises a mesh or aids in mixing when the collapsible blood container **102** is agitated.

The present disclosure provides for, and includes, an oxygen depletion device having an outer receptacle **101** that is substantially impermeable to oxygen enclosing an inner collapsible blood container **102** and providing a headspace. In an aspect, the oxygen sorbent **103** is disposed within the headspace thereby creating and an oxygen depleted state within the headspace. In an aspect, said oxygen sorbent **103** disposed in the headspace further maintains the headspace in an oxygen depleted state by removing oxygen that may enter through the outer receptacle **101** or through the one or more inlets/outlets **30.**

Maintaining the headspace in an oxygen depleted state provides for improved shelf life for oxygen depletion device **10.** In an aspect, the shelf life of an assembled oxygen depletion device **10** has a shelf life of at least 24 months. In another aspect, the oxygen depletion device **10** has a shelf life of at least 12 months after assembly of the components. In an aspect according to the present disclosure, the assembled oxygen depletion device **10** meets ISTA-2A standards.

In certain aspects of the present disclosure, the headspace provides for improved processing times. For oxygen depletion device **10,** removing ambient air present or inert flushing gas from the assembly prior to sealing the outer receptacle **101** reduces the volume of the headspace. Applying a vacuum to the outer receptacle **101** prior to sealing reduces the volume of the headspace and decreases the total volume of the assembled oxygen depletion device. While reduced overall headspace volume provides for reduced shipping volume, it can result in increased filling times by constraining the collapsible blood container **102.** In certain aspects, the headspace may be flushed with nitrogen gas and then sealed under slightly less than ambient pressure to provide a reduced headspace volume in the oxygen depletion device **10** without significantly increasing the fill and process time.

In certain aspects, the headspace may be initially depleted of oxygen by flushing the headspace with nitrogen. In an aspect, the headspace of oxygen depletion device **10** is flushed with nitrogen gas prior to sealing the outer receptacle **101.** In an aspect, the flushing gas is ≥99.9% nitrogen gas.

The present disclosure includes and provides for oxygen depletion device **10** having inner collapsible blood container **102** divided into two or more compartments. In certain aspects, an oxygen depletion device **10,** having a collapsible blood container **102** divided into multiple compartments has a headspace of between 10 and 500 ml per compartment. In an aspect the headspace is between 20 and 400 ml per compartment. In another aspect the headspace volume is between 60 and 300 ml per compartment. In a further aspect, the headspace volume is between 100 and 200 ml per compartment of a collapsible blood container. In an aspect, an oxygen depletion device **10** having inner collapsible blood container **102** divided into compartments has a headspace of about 10 ml per compartment. In another aspect, the headspace is about 100 ml to about 200 ml per compartment. In another aspect the headspace is about 300 ml to about 500 ml per compartment.

The present disclosure includes and provides for oxygen depletion device 10 having inner collapsible blood container **102** divided into two or more compartments. In certain aspects, an oxygen depletion device 10, having a collapsible blood container **102** divided into two compartments has a headspace of between 20 and 1000 ml. In an aspect the headspace is between 100 and 800 ml. In another aspect the headspace volume is between 200 and 700 ml. In a further aspect, the headspace volume is between 300 and 500 ml for a two compartment collapsible blood container. In an aspect, an oxygen depletion device **10** having inner collapsible blood container **102** divided into two compartments has a headspace of about 700 ml. In another aspect, the headspace is about 200 ml to about 700 ml. In another aspect the headspace is about 300 ml to about 500 ml.

The present disclosure includes and provides for oxygen depletion device **10** having inner collapsible blood container **102** divided into two or more compartments. In certain aspects, an oxygen depletion device **10,** having a collapsible blood container **102** divided into three compartments has a headspace of between 20 and 1000 ml. In an aspect the headspace is between 100 and 800 ml. In another aspect the headspace volume is between 200 and 700 ml. In a further aspect, the headspace volume is between 400 and 600 ml for a three compartment collapsible blood container. In an aspect, an oxygen depletion device **10** having inner collapsible blood container **102** divided into three compartments has a headspace of about 800 ml. In another aspect, the headspace is about 200 ml to about 700 ml. In another aspect the headspace is about 400 ml to about 600 ml.

In accordance with the present invention, an oxygen depletion device 10 is provided having an inner collapsible blood container **102** and further including one or more spacers **110** that ensure the separation of the outer receptacle **101** and the inner collapsible blood container **102.** The spacer **110** provides for the maintenance of the headspace in the oxygen depletion device to ensure efficient diffusion of the oxygen from the surface of membrane **113** to the sorbent **103.** A spacer **110** can be prepared from one or more of the materials selected from the group consisting of a mesh, a molded mat, a woven mat, a non-woven mat, a strand veil, and a strand mat. In certain aspects, the spacer **110** can be integrated directly into the collapsible blood container **102** as ribs, dimples, or other raised feature that maintains a separation between the outer receptacle **101** and the inner collapsible blood container **102.** The present specification also includes and provides for a spacer **110** to be integrated into the outer receptacle **101** as ribs, dimples, or other suitable raised feature capable of maintaining a separation between the outer receptacle **101** and the inner collapsible blood container **102.**

The present disclosure also includes and provides for inner collapsible blood containers **102** that further comprise a window **112.** As used herein, a window **112** is made of a transparent material and is bonded or otherwise incorporated into the inner collapsible blood container **102.** In accordance with the present disclosure, suitable materials for window **112** are blood compatible. In certain aspects, materials suitable for a window **112** are oxygen impermeable. In other aspects, materials suitable for a window **112** are oxygen impermeable. The size of a window **112** need only be large enough to provide observation of the blood.

Also included and provided for by the present disclosure are collapsible blood containers having bis(2-ethylhexyl) phthalate (DEHP). DEHP is included in most PVC based blood storage bags as a plasticizer where it has been observed that DEHP provides a protective effect to stored red blood cells. See U.S. Patent 4,386,069 issued to Estep. In certain aspects, an oxygen depletion device **10** may further include DEHP incorporated in the inner collapsible blood container **102.** In other aspects, DEHP may be provided separately within the inner collapsible blood container **102.**

The present disclosure provides for, and includes, an oxygen depletion device **10** that does not include DEHP. It has been hypothesized that DEHP may act as an endocrine disruptor and certain regulatory agencies are considering ordering the removal of DEHP from blood bags. It has been observed that DEHP may not be necessary when red blood cells are stored anaerobically. *See,* International Patent Publication No. WO 2014/134503. Accordingly, in certain aspects, oxygen depletion device **10** entirely excludes DEHP from all blood contacting surfaces. In other aspects, oxygen depletion device **10** limits DEHP containing surfaces to tubing, ports, and inlets such as those illustrated in the Figures at, for example, **106** and **205.** In an aspect, oxygen depletion device **10** excludes a DEHP containing collapsible blood container **102.**

The present disclosure also includes and provides for inner collapsible blood containers **102** that include an inlet/outlet **130.** As provided below, an inlet/outlet **130** may be incorporated into the inner collapsible blood containers **102** during assembly using a frame **120** or may be integrated into the inner collapsible blood container **102** during manufacture using compression or blow molding. An inlet/outlet **130** may comprise silicone. In other aspects, inlet/outlet **130** may comprise materials selected from the group consisting of ethylene-vinyl acetate (EVA), poly(ethylene-vinyl) acetate (PEVA), polypropylene (PP), polyurethane (PU), polyester (PES), polyethylene terephthalate (PET), polyethylene (PE), high-density polyethylene (HDPE), polyvinyl chloride (PVC), polyvinylidene chloride (PVDC), low-density polyethylene (LDPE), polypropylene (PP), polystyrene (PS), high impact polystyrene (HIPS), polyamides (PA) (*e.g.,* nylon), acrylonitrile butadiene styrene (ABS), polycarbonate (PC), polycarbonate/acrylonitrile butadiene styrene (PC/ABS), polyurethanes (PU), melamine formaldehyde (MF), plastarch material, phenolics (PF), polyetheretherketone (PEEK), polyetherimide (PEI) (Ultem), polylactic acid (PLA), polymethyl methacrylate (PMMA), polytetrafluoroethylene (PTFE), urea-formaldehyde, ethylene vinyl alcohol copolymer (EVOH), and polyamide.

As used herein, the term "blood" refers to whole blood, leukoreduced RBCs, platelet reduced RBCs, and leukocyte and platelet reduced RBCs. The term blood further includes packed red blood cells, platelet reduced packed red blood cells, leukocyte reduced packed red blood cells (LRpRBC), and leukocyte and platelet reduced packed red blood cells. The temperature of blood can vary depending on the stage of the collection process, starting at the normal body temperature of 37 °C at the time and point of collection, but decreasing rapidly to about 30 °C as soon as the blood leaves the patient's body and further thereafter to room temperature in about 6 hours when untreated, and ultimately being refrigerated at between about 4 °C and 6 °C.

As used herein, the term "whole blood" refers to a suspension of blood cells that contains red blood cells (RBCs), white blood cells (WBCs), platelets suspended in plasma, and includes electrolytes, hormones, vitamins, antibodies, etc. In whole blood, white blood cells are normally present in the range between 4.5 and 11.0 x 10⁹ cells/L and the normal RBC range at sea level is 4.6-6.2 x 10¹²/L for men and 4.2-5.4 x 10¹²/L for women. The normal hematocrit, or percent packed cell volume, is about 40-54% for men and about 38-47% for women. The platelet count is normally 150-450 x 10⁹/L for both men and women. Whole blood is collected from a blood donor, and is usually combined with an anticoagulant. Whole blood, when collected is initially at about 37 °C and rapidly cools to about 30 °C during and shortly after collection, but slowly cools to ambient temperature over about 6 hours. Whole blood may be processed according to methods of the present disclosure at collection, beginning at 30-37 °C, or at room temperature (typically about 25 °C). As used herein, a "unit" of blood is about 450-500 ml including anticoagulant.

As used herein, "red blood cells" (RBCs) includes RBCs present in whole blood, leukoreduced RBCs, platelet reduced RBCs, and leukocyte and platelet reduced RBCs. Human red blood cells *in vivo* are in a dynamic state. The red blood cells contain hemoglobin, the iron-containing protein that carries oxygen throughout the body and gives red blood its color. The percentage of blood volume composed of red blood cells is called the hematocrit. As used herein, unless otherwise limited, RBCs also includes packed red blood cells (pRBCs). Packed red blood cells are prepared from whole blood using centrifugation techniques commonly known in the art. As used herein, unless otherwise indicated, the hematocrit of pRBCs is about 50%.

The present disclosure provides for, and includes, a blood storage device **20,** for storing oxygen depleted blood and maintaining the blood in a deoxygenated state during the storage period. Certain anaerobic blood storage devices (ASB) are known in the art, including for example U.S. Patent No. 6,162,396 to Bitensky et al. The anaerobic blood storage devices of the prior art did not include ports and inlets designed to be substantially impermeable to oxygen. Accordingly, the prior art anaerobic storage devices had poor shelf lives prior to use and were susceptible to significant ingress of oxygen. As provided in the present disclosure, an improved blood storage device **20** comprising features directed to maintaining the integrity of the device while allowing for the sampling of the blood that occurs during storage and blood banking. The improved ASB also provides for improved diffusion of oxygen from the blood, providing for additional depletion during the storage period.

The blood storage device **20** comprises an outer receptacle **201** that is substantially impermeable to oxygen, a collapsible blood container **202** comprising a locating feature **203** adapted to align the collapsible blood container **202** within the geometry of the outer receptacle **201;** at least one inlet/outlet **30** comprising connecting to the collapsible blood container **202** and a bond **206** to the outer receptacle **201,** wherein the bond **206** to the outer receptacle **201** is substantially impermeable to oxygen and an oxygen sorbent **207** situated within the outer receptacle **201.**

As used herein, an outer receptacle **201** is at least equivalent to an outer receptacle **101.** Also as used herein, an inner collapsible blood container **202** includes blood containers as provided above for an inner collapsible blood container **102** but also provides for collapsible blood containers **202** comprising materials that are less permeable to oxygen, such as PVC. Also as provided herein, oxygen sorbent **207** is at least equivalent to sorbent **103** and may be provided in sachets as discussed above.

Like a reinforced membrane **600,** a reinforced membrane **700** comprises a silicone membrane **113** that is substantially permeable to oxygen and reinforced with a features **701.** Reinforced membranes **700** are suitable for the preparation of inner collapsible containers **102** for use in oxygen depletion devices **10.** In certain aspects, the reinforced silicone membranes **700** may be further characterized as having a relatively smooth surface for contact with the blood or blood component for depletion. In other aspects, the blood contacting surface can be modified with additional features to provide for additional mixing. In certain aspects, the features **701** of a membrane **700** can be presented for contact with the blood in an inner collapsible container **102** to improve mixing.

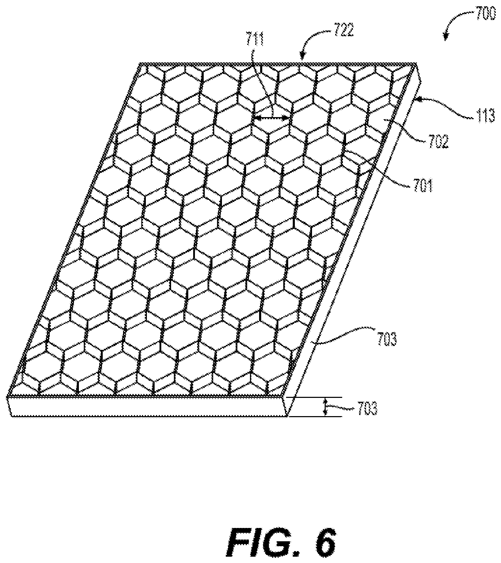

The present disclosure provides for, and includes a membrane **700** that is illustrated generally by way of example, but not by way of limitation, as shown in Figure 6. More specifically, Figure 6 presents an aspect of a membrane **700** having raised features **701** arranged in a pattern **722.** As will be provided below, the arrangement of raised features **701** can be formed in a variety of patterns **720.** Referring to Figure 6, the general aspects of a reinforced silicone membrane **700** comprises a silicone membrane **113** having an area **702** having an average thickness **703** of less than 100 x 10⁻⁶ M (µm), and features **701.** Like reinforced membranes **600,** reinforced membranes **700** provide for the manufacture of inner collapsible blood containers **102** that may help comply with ISO standard 3826-1:2013 that requires that plastic collapsible blood containers shall not show leakage when placed between two plates and subjected to an internal pressure of 50 kPa above atmospheric pressure for 10 minutes. Reinforced membranes **700** overcome the strength limitations of silicone membranes **113** when the thickness is reduced to less than 100 µm. As provided herein, reinforced membranes **700** provide for a silicone membrane **113** has a thickness **703** of between 5 and 100 µm.

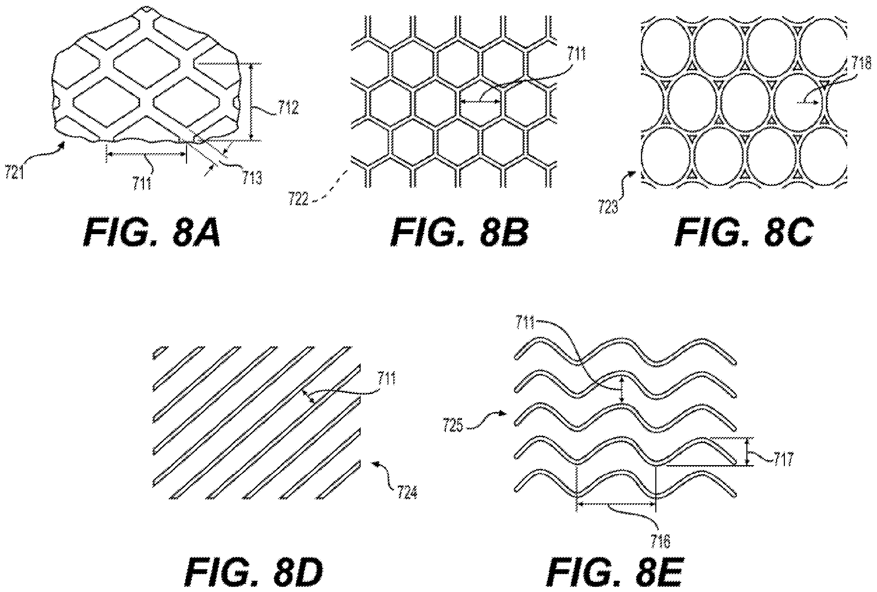

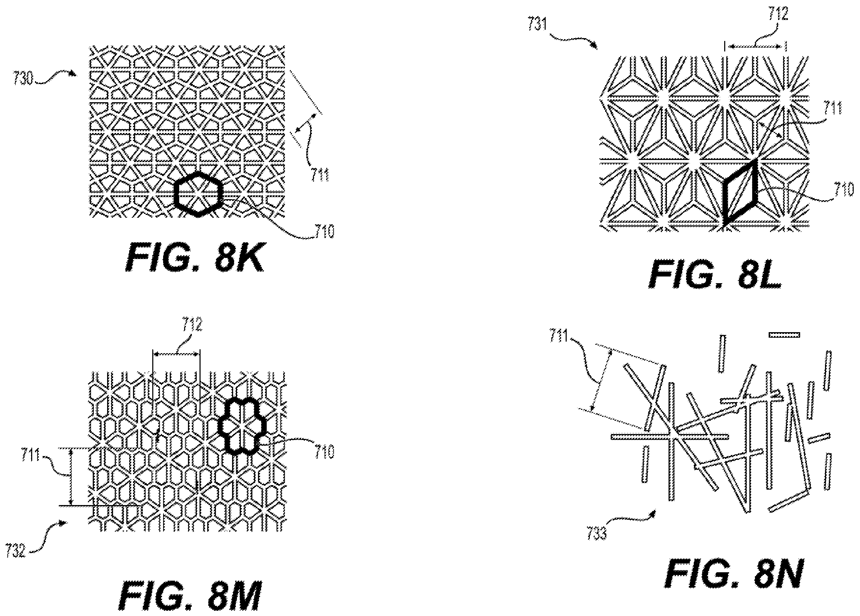

Referring to Figure 6, the raised features **701** are arranged in a pattern **722** (hexagonal) that encloses an area **702.** As used herein, area **702** refers to the areas of silicone membrane **113** that are less than 100 µm in average thickness (*e.g*., thickness **703).** While shown in Figure 6 as being surrounded by features **701,** in aspects according to the present specification, area **702** does not need to be surrounded by raised features. As will be discussed below, the raised features **701** may be arranged as raised lines (*e.g*., Figure 8D, pattern **724),** waved lines (*e.g.,* Figure 8E, pattern **724),** or random features (*e.g.,* Figure 8N, pattern **733).** Accordingly, area **702** refers to those areas of membrane **700** that are less than 100 µm and are not areas having features **701.**

In aspects of the present disclosure, silicone membrane **113** has an average thickness **703** of less than 100 x 10⁻⁶ M (µm) in areas **702.** As used herein, the thickness **703** refers to the average thickness of area **702.** In aspects of the present disclosure, the average thickness **703** of area **702** can vary ± 10%. In certain aspects, silicone membrane **113** has an average thickness **703** of 50 µm. In some aspects, silicone membrane **113** has an average thickness **703** of 40 µm. In another aspect, silicone membrane **113** has an average thickness **703** of 30 µm. In certain aspects, silicone membrane **113** has an average thickness **703** of 25 µm. In other aspects, silicone membrane **113** has an average thickness **703** of 20 µm. In certain aspects, silicone membrane **113** has an average thickness **703** of less than 50 µm. In some aspects, silicone membrane **113** has an average thickness **703** of less than 40 µm. In other aspects, silicone membrane **113** has an average thickness **703** of less than 30 µm. In yet other aspects, silicone membrane **113** has an average thickness **703** of less than 20 µm. In certain aspects, silicone membrane **113** has an average thickness **703** of between 5 µm and 95 µm. In other aspects, silicone membrane **113** has an average thickness **703** of between 20 µm and 95 µm. In other aspects, silicone membrane **113** has an average thickness **703** of between 5 µm and 50 µm. In a further aspect, silicone membrane **113** has an average thickness **703** of between 5 µm and 20 µm. In yet other aspects, silicone membrane **113** has an average thickness **703** of between 10 µm and 40 µm. In certain aspects, silicone membrane **113** has an average thickness **703** of between 10 µm and 30 µm. In yet other aspects, silicone membrane **113** has an average thickness **703** of between 10 µm and 25 µm. In a further aspect, silicone membrane **113** has an average thickness **703** of between 15 µm and 35 µm.

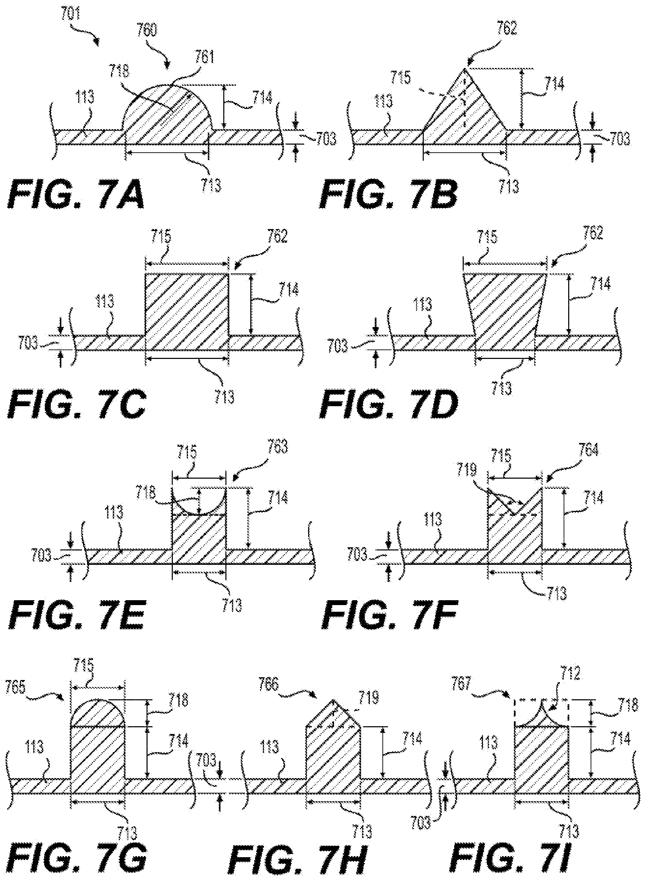

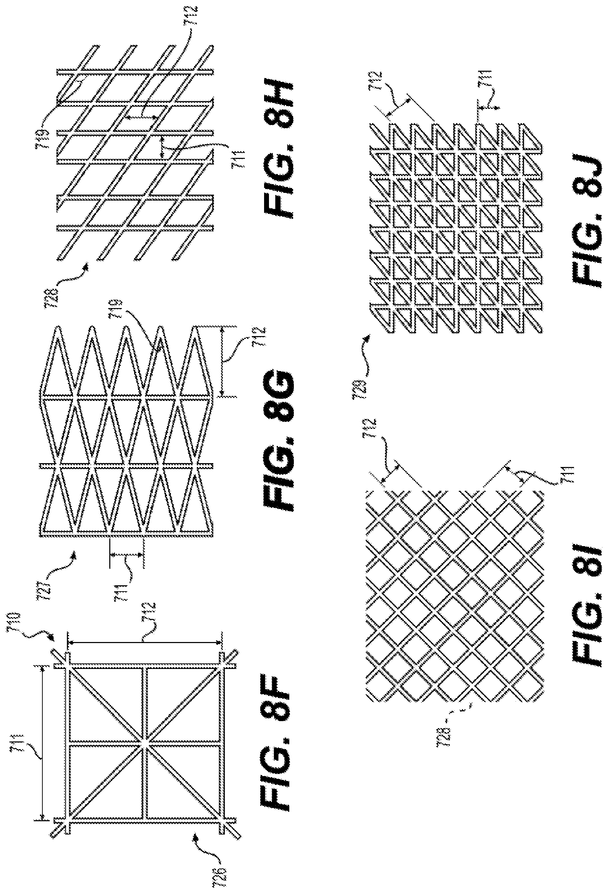

The present disclosure provides for and includes, a reinforced membrane **700,** as illustrated in Figure 6, having features **701** that reinforce the silicone membrane **113.** Features **701** provide for mechanical support and strengthen the membrane to reduce tearing splitting, or rupturing when the reinforced membrane **700** is used to prepare a collapsible blood container **102.** More specifically, the features **701** provide for reinforcement of the silicone membrane **113** and allow for improved structural integrity when devices made with the reinforced membrane **700** are subjected to conditions routinely found, for example, in a blood collection center. These conditions include, for example, stacking, mixing, or centrifuging of filled collapsible blood containers **102.** Importantly, the features **701** of a reinforced membrane **700** provide for improved durability and allow for collapsible blood containers **102** prepared therefrom to survive drop tests. As shown in Figure 7, feature **701** can be prepared having a variety of cross sections **760.** As shown in Figure 8, the features **701** having cross sections **760** can be applied to, or incorporated in, silicone membrane **113** in a variety of patterns **720.** Alternatively, as shown in Figure 8, the features **701** having cross sections **760** can be applied to, or incorporated in, silicone membrane **113** randomly as shown for example in Figure 8N, pattern **733.**

In aspects of the present disclosure, the features **701** may be disposed on one side of membrane **113** or on both sides of silicone membrane **113.** In an aspect of the present disclosure, features **701** are disposed on one side of silicone membrane **113.** In another aspect of the present disclosure, features **701** are disposed on both sides of the silicone membrane **113.** As provided herein, features **701** disposed on one side of silicone membrane **113** may be different than features **701** disposed on the opposite side of silicone membrane **113.**

As will be appreciated, less than the entire surface of silicone membrane **113** is covered by features **701.** More specifically, in order to retain the desirable oxygen permeability of a reinforced membrane **700,** the area **702** having a thickness **703** of less than 100 µm should be maximized. Similarly, the cross section **760** and the pattern **720** are selected to, among other criteria, maximize the strength of the reinforced membrane **700.** In other aspects, the cross section **760** and the pattern **720** may be selected to improve mixing of blood in a collapsible blood container **102.** In yet other aspects, the cross section **760** and the pattern **720** may be selected to improve diffusion of oxygen in the headspace of an oxygen depletion device **10** whereby the features **701** function as a spacer **110,** or a spacer **213** of a blood storage device **20.**

The present disclosure provides for, and includes, a reinforced membrane **700** having features **701** covering a percentage of less than 50% of the area of at least one side of silicone membrane **113.** That is, features **701,** having a width of length **713** and a height of length **714** and cover an area of silicone membrane **113** of less than 50% such that 50% of silicone membrane **113** comprises area **702** having an average thickness **703** of less than 100 µm. The present disclosure provides for, and includes, a reinforced membrane **700** having features **701** covering a percentage of less than 30% of the area of at least one side of silicone membrane **113.** That is, features **701,** having a width of length **713** and a height of length **714** and cover an area of silicone membrane **113** of less than 50% such that 50% of silicone membrane **113** comprises area **702** having an average thickness **703** of less than 100 µm. In an aspect, features **701** covers between 30 and 50% of the area of at least one side of silicone membrane **113.** In an aspect, features **701** covers between 10 and 50% of the area of at least one side of silicone membrane **113.** In an aspect, features **701** covers between 10 and 30% of the area of at least one side of silicone membrane **113.** In an aspect, features **701** covers between 20 and 40% of the area of at least one side of silicone membrane **113.** In another aspect, features **701** covers between 0.5 and 10% of the area of at least one side of silicone membrane **113.** In another aspect, features **701** covers between 5.0 and 10% of the area of at least one side of silicone membrane **113.** In another aspect, features **701** covers between 10 and 25% of the area of at least one side of silicone membrane **113.** In a further aspect, feature **701** covers at least 1% of the area of at least one side of silicone membrane **113.** In a further aspect, feature **701** covers at least 5% of the area of at least one side of silicone membrane **113.** As provided herein, features **701,** covering a percentage of less than 30% of the area of silicone membrane **113** are arranged in a pattern **720.** In aspects of the present disclosure, features **701,** covering a percentage of less than 30% of the area of silicone membrane **113** are arranged in a pattern selected from the group consisting of pattern **721,** pattern **722,** pattern **723,** pattern **724,** pattern **725,** pattern **726,** pattern **727,** pattern **728,** pattern **729,** pattern **730,** pattern **731,** pattern **732,** and pattern **733,** as illustrated in Figure 8. As provided herein, features **701,** covering a percentage of less than 50% of the area of silicone membrane **113** are arranged in a pattern **720.** In aspects of the present disclosure, features **701,** covering a percentage of less than 50% of the area of silicone membrane **113** are arranged in a pattern selected from the group consisting of pattern **721,** pattern **722,** pattern **723,** pattern **724,** pattern **725,** pattern **726,** pattern **727,** pattern **728,** pattern **729,** pattern **730,** pattern **731,** pattern **732,** and pattern **733,** as illustrated in Figure 8.

The present disclosure provides for and includes, a reinforced membrane **700,** having silicone membrane **113** with an average thickness **703** of less than 100 x 10⁻⁶ M (µm) in area **702,** reinforced with features **701** with a cross-section **760,** as illustrated in Figure 7. In aspects of the present disclosure, features **701** with cross-section **760** can have a pattern **720** selected from the group consisting of patterns **721** to **732,** and **733,** as illustrated in Figure 8. The present disclosure provides for features **701** having a cross-section **760** selected from the group consisting of **761, 762, 763, 764, 765, 766,** and **767,** as illustrated in Figure 7. As provided herein, features **701** have a cross-section **760** having a length **713** perpendicular to length **714.** In certain aspects, features **701** have cross-section **760** further comprise a length **715** perpendicular to a length **714.** In other aspects, features **701** have cross-section **760** having a length **713** perpendicular to length **714** and further comprise a radius of length **718.** In a further aspect, features **701** have cross-section **760** having a length **713** perpendicular to length **714,** and further comprise a length **715** perpendicular to a length **714** and a radius of length **718.** In another aspect, features **701** have cross-section **760** having a length **713** perpendicular to length **714,** a length **715** perpendicular to a length **714,** and an angle **719.**

The present disclosure provides for and includes, a reinforced membrane **700,** having silicone membrane **113** with an average thickness **703** of less than 100 x 10⁻⁶ M (µm) in areas **702,** reinforced with features **701** with a cross-section **760,** as illustrated in Figure 7. In aspects of the present disclosure, features **701** with cross-section **760** can have a pattern **720** selected from the group consisting of patterns **721** to **732,** and **733,** as illustrated in Figure 8. The present disclosure provides for features 701 having a cross-section **760** selected from the group consisting of **761, 762, 763, 764, 765, 766,** and **767,** as illustrated in Figure 7. As provided herein, features **701** have cross-section **760** having a length **713** of less than 2500 µm, perpendicular to length **714** of less than 2500 µm. In certain aspects, features **701** have cross-section **760** further comprise a length **715** of less than 2500 µm, perpendicular to a length **714** of less than 2500 µm. In other aspects, features **701** have cross-section **760** having a length **713** of less than 2500 µm, perpendicular to length **714** of less than 2500 µm, and further comprise a radius of length **718** of less than 1250 µm. In a further aspect, features **701** have cross-section **760** having a length **713** of less than 2500 µm, perpendicular to length **714** of less than 2500 µm, and further comprise a length **715** of less than 2500 µm, perpendicular to a length **714** and a radius of length **718** of less than 1250 µm. In another aspect, features **701** have cross-section **760** having a length **713** of less than 2500 µm, perpendicular to length **714** of less than 2500 µm, a length **715** of less than 2500 µm, perpendicular to a length **714** of less than 2500 µm, and an angle **719** of less than 60 °.

In an aspect of the present disclosure, reinforced membrane **700,** has a silicone membrane **113** having a thickness **703** of less than 100 µm and features **701** having a cross-section **761,** with a length **714** of less than 2500 µm, and a radius of length **718** of less than 1250 µm. In certain aspects, features **701** having a cross-section **761,** with a length **714** of less than 2500 µm, and a radius of length **718** of less than 100 µm. In certain aspects, features **701** have a cross-section **761** having a length **714** of between 250 µm and 1000 µm, and a radius of length **718** of between 125 µm and 500 µm. In other aspects, features **701** have a cross-section **761** having a length **714** of between 100 µm and 500 µm, and a radius of length **718** of between 50 µm and 250 µm. In certain aspects, features **701** have a cross-section **761** having a length **714** of between 100 µm and 2500 µm, and a radius of length **718** of between 50 µm and 1250 µm. In other aspects, features **701** have a cross-section **761** having a length **714** greater than a radius of length **718.** In yet other aspects, features **701** have a cross-section **761** having a length **714** less than a radius of length **718.** In certain aspects, features **701** have a cross-section **761** having a length **714** of between 100 and 2500 µm. In certain aspects, features **701** have a cross-section **761** having a radius of length **718** between 5 µm and 100 µm.

In an aspect of the present disclosure, reinforced membrane **700,** has a silicone membrane **113** having a thickness **703** of less than 100 µm and features **701** having a cross-section **762,** with a length **713** of less than 2500 µm, a length **714** of less than 2500 µm and a length **715** of less than 2500 µm. In certain aspects, features **701** have a cross-section **762,** with a length **713** of less than 1000 µm, a length **714** of less than 1000 µm and a length **715** of less than 1000 µm. In certain aspects, features **701** have a cross-section **762,** with a length **713** of less than 500 µm, a length **714** of less than 500 µm and a length **715** of less than 500 µm. In other aspects, features **701** have a cross-section **762,** with a length **713** of between 100 µm and 2500 µm, a length **714** of between 100 µm and 2500 µm, and a length **715** of between 100 µm and 2500 µm. In other aspects, features **701** have a cross-section **762,** with a length **713** of between 250 µm and 1000 µm, a length **714** of between 250 µm and 1000 µm, and a length **715** of between 250 µm and 1000 µm. In yet other aspects, features **701** have a cross-section **762,** with a length **713** of between 250 µm and 1000 µm, a length **714** of between 250 µm and 1000 µm, and a length **715** equal to length **713.** In a further aspect, features **701** have a cross-section **762,** with a length **713** of between 250 µm and 1000 µm, a length **714** of between 250 µm and 1000 µm, and a length **715** less than length **713.** In a further aspect, features **701** have a cross-section **762,** with a length **713** of between 250 µm and 1000 µm, a length **714** of between 250 µm and 1000 µm, and a length **715** greater than length **713.** In a further aspect, features **701** have a cross-section **762,** with a length **713** of between 250 µm and 1000 µm, a length **714** of between 250 µm and 1000 µm, and a length **715** equal to zero. In aspects of the present disclosure, reinforced membrane **700,** has a silicone membrane **113** having a thickness **703** of less than 100 µm and features **701** having a cross-section **762,** wherein all corners can be curves.

In an aspect of the present disclosure, reinforced membrane **700,** has a silicone membrane **113** having a thickness **703** of less than 100 µm and features **701** having a cross-section **763,** with a length **713** of less than 2500 µm, a length **714** of less than 2500 µm, a length **715** of less than 2500 µm, and a radius of length **718** of less than 1250. In certain aspects, features **701** have a cross-section **763,** with a length **713** of less than 1000 µm, a length **714** of less than 1000 µm, a length **715** of less than 1000 µm, and a radius of length **718** of less than 500. In certain aspects, features **701** have a cross-section **763,** with a length **713** of less than 500 µm, a length **714** of less than 500 µm, a length **715** of less than 500 µm, and a radius of length **718** of less than 250 µm. In other aspects, features **701** have a cross-section **763,** with a length **713** of between 100 µm and 2500 µm, a length **714** of between 100 µm and 2500 µm, a length **715** of between 100 µm and 2500 µm, and a radius of length **718** of less than length **714.** In yet other aspects, features **701** have a cross-section **763,** with a length **713** of between 100 µm and 2500 µm, a length **714** of between 100 µm and 2500 µm, a length **715** of between 100 µm and 2500 µm, and a radius of length **718** equal to length **714.** In other aspects, features **701** have a cross-section **763,** with a length **713** of between 250 µm and 1000 µm, a length **714** of between 250 µm and 1000 µm, a length **715** of between 250 µm and 1000 µm, and a radius of length **718** of between 5 and 1000 µm. In yet other aspects, features **701** have a cross-section **763,** with a length **713** of between 250 µm and 1000 µm, a length **714** of between 250 µm and 1000 µm, a length **715** equal to length **713** and a radius of length **718** of between 5 and 1000 µm. In a further aspect, features **701** have a cross-section **763,** with a length **713** of between 250 µm and 1000 µm, a length **714** of between 250 µm and 1000 µm, a length **715** less than length **713,** and a radius of length **718** of between 5 and 1000 µm.

In an aspect of the present disclosure, reinforced membrane **700,** has a silicone membrane **113** having a thickness **703** of less than 100 µm and features **701** having a cross-section **764,** with a length **713** of less than 2500 µm, a length **714** of less than 2500 µm, a length **715** of less than 2500 µm, and an angle **719** of less than 60 °. In certain aspects, features **701** have a cross-section **764,** with a length **713** of less than 1000, a length **714** of less than 1000 µm, a length **715** of less than 1000 µm, and an angle **719** of less than 60 °. In certain aspects, features **701** have a cross-section **764,** with a length **713** of less than 500 µm, a length **714** of less than 500 µm, a length **715** of less than 500 µm, and angle **719** of less than 60 °. In other aspects, features **701** have a cross-section **764,** with a length **713** of between 100 µm and 2500 µm, a length **714** of between 100 µm and 2500 µm, a length **715** of between 100 µm and 2500 µm, and an angle **719** of between 20° and 60°. In other aspects, features **701** have a cross-section **764,** with a length **713** of between 250 µm and 1000 µm, a length **714** of between 250 µm and 1000 µm, a length **715** of between 250 µm and 1000 µm, and an angle **719** of between 20° and 60°.

In an aspect of the present disclosure, reinforced membrane **700,** has a silicone membrane **113** having a thickness **703** of less than 100 µm and features **701** having a cross-section **765,** with a length **713** of less than 2500 µm, a length **714** of less than 2500 µm, a length **715** of less than 2500 µm, and a radius of length **718** of less than 1250 µm. In certain aspects, features **701** have a cross-section **765,** with a length **713** of less than 1000 µm, a length **714** of less than 1000 µm, a length **715** of less than 1000 µm, and a radius of length **718** of less than 500. In certain aspects, features **701** have a cross-section **765,** with a length **713** of less than 500, a length **714** of less than 500 µm, a length **715** of less than 500 µm, and a radius of length **718** of less than 250 µm. In other aspects, features **701** have a cross-section **765,** with a length **713** of between 100 µm and 2500 µm, a length **714** of between 100 µm and 2500 µm, a length **715** of between 100 µm and 2500 µm, and a radius of length **718** of less than length **714.** In yet other aspects, features **701** have a cross-section **765,** with a length **713** of between 100 µm and 2500 µm, a length **714** of between 100 µm and 2500 µm, a length **715** of between 100 µm and 2500 µm, and a radius of length **718** equal to length **714.** In other aspects, features **701** have a cross-section **765,** with a length **713** of between 250 µm and 1000 µm, a length **714** of between 250 µm and 1000 µm, a length **715** of between 250 µm and 1000 µm, and a radius of length **718** of between 5 and 500 µm. In yet other aspects, features **701** have a cross-section **765,** with a length **713** of between 250 µm and 1000 µm, a length **714** of between 250 µm and 1000 µm, a length **715** equal to length **713** and a radius of length **718** of between 5 and 500 µm. In a further aspect, features **701** have a cross-section **765,** with a length **713** of between 250 µm and 1000 µm, a length **714** of between 250 µm and 1000 µm, a length **715** less than length **713,** and a radius of length **718** of between 5 and 500 µm.

In an aspect of the present disclosure, reinforced membrane **700,** has a silicone membrane **113** having a thickness **703** of less than 100 µm and features **701** having a cross-section **766,** with a length **713** of less than 2500 µm, a length **714** of less than 2500 µm, and an angle **719** of less than 60 °. In certain aspects, features **701** have a cross-section **766,** with a length **713** of less than 1000 µm, a length **714** of less than 1000 µm, and an angle **719** of less than 60 °. In certain aspects, features **701** have a cross-section **766,** with a length **713** of less than 500, a length **714** of less than 500 µm, and angle **719** of less than 60 °. In other aspects, features **701** have a cross-section **766,** with a length **713** of between 100 µm and 2500 µm, a length **714** of between 100 µm and 2500 µm, and an angle **719** of between 20° and 60°. In other aspects, features **701** have a cross-section **766,** with a length **713** of between 250 µm and 1000 µm, a length **714** of between 250 µm and 1000 µm, and an angle **719** of between 20° and 60°. In yet other aspects, features **701** have a cross-section **766,** with a length **713** of between 250 µm and 1000 µm, a length **714** of between 250 µm and 1000 µm, an angle **719** of between 20° and 60°, and a length **715** of less than the length **713.**

In an aspect of the present disclosure, reinforced membrane **700,** has a silicone membrane **113** having a thickness **703** of less than 100 µm and features **701** having a cross-section **767,** with a length **713** of less than 2500 µm, a length **714** of less than 2500 µm, a length **715** less than length **713,** and two radii of length **718** of less than 1250 µm each. In certain aspects, features **701** have a cross-section **767,** with a length **713** of less than 1000, a length **714** of less than 1000 µm, a length **715** less than length **713,** and two radii of length **718** of less than 500 µm each. In certain aspects, features **701** have a cross-section **767,** with a length **713** of less than 500 µm, a length **714** of less than 500 µm, a length **715** less than length **713,** and two radii of length **718** of less than 250 µm each. In other aspects, features **701** have a cross-section **767,** with a length **713** of between 100 µm and 2500 µm, a length **714** of between 100 µm and 2500 µm, a length **715** less than length **713,** and two radii of length **718** of less than length **714.**

The present disclosure provides for and includes, a reinforced membrane **700,** having a feature **701** with length **713** of less than 10000 µm, perpendicular to length **714** of less than 5000 µm. In certain aspects, feature **701** has a length **713** of less than 2500 µm, perpendicular to length **714** of less than 2500 µm. In other aspects, feature **701** has a length **713** of less than 1000 µm, perpendicular to length **714** of less than 1000 µm. In other aspects, feature **701** has a length **713** of less than 500 µm, perpendicular to length **714** of less than 500 µm. In another aspect, feature **701** has a length **713** of between 200 and 5000 µm, perpendicular to length **714** of between 20 and 5000 µm. In a further aspect, feature **701** has a length **713** of between 300 and 800 µm, perpendicular to length **714** of between 20 and 5000 µm. In another aspect, feature **701** has a length **713** of between 1000 and 5000 µm, perpendicular to length **714** of between 20 and 5000 µm. In certain aspects, feature **701** has a length **713** of between 5000 and 8000 µm, perpendicular to length **714** of between 20 and 5000 µm. In other aspects, feature **701** has a length **713** of between 500 and 2500 µm, perpendicular to length **714** of between 20 and 5000 µm. In yet another aspect, feature **701** has a length **713** selected from the group consisting of between 200 and 5000 µm, between 300 and 800 µm, between 1000 and 5000 µm, and between 5000 and 8000 µm, perpendicular to length **714** selected from the group consisting of between 20 and 100 µm, between 20 and 5000 µm, between 100 and 2000 µm, between 100 and 500 µm, between 500 and 1000 µm, 1500 and 2000 µm, between 2000 and 3500 µm, and between 3500 and 5000 µm. In yet other aspects, feature **701** has a length **713,** perpendicular to length **714,** wherein length **713** is equal to length **714.** In certain aspects, feature **701** has a length **713,** perpendicular to length **714,** wherein length **713** is greater than length **714.** In certain aspects, feature **701** has a length **713,** perpendicular to length **714,** wherein length **713** is less than length **714.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **720.** Non-limiting examples of selected patterns suitable for use in a reinforced membrane **700** are illustrated in Figure 8. As patterns for tiling a Euclidian plane are known in the art, it is understood that other patterns **720** may be incorporated into a reinforced membrane **700** wherein the area **702** comprises at least 70% of the surface and the features **701** comprise 30% or less of the surface. In aspects according to the present disclosure, pattern **720** is selected from the group consisting of patterns **721** to **732,** and **733,** as illustrated in Figure 8. As noted above, features **701** provide for mechanical support and strengthen the membrane to reduce tearing splitting, or rupturing. In some aspects, the pattern **720** is a regular tiling pattern, for example patterns **721, 722, 728,** or **730,** and as illustrated at Figure 8A, 8B, 8H, and 8K. Regular tiling patterns having regular polygons that are all the same (e.g., triangle, square, hexagon) are known in the art. In other aspects, pattern **720** is a uniform tiling (also known as a semi-regular tiling) that comprises a mixture of different regular polygons. Uniform tilings are well known in the art. In yet other aspects, the pattern **720** is a non-regular, non-uniform pattern, for example as illustrated in Figure 8N, pattern **733.** As will be appreciated by a person of skill in the art, an very large number of possible patterns **720** are available for use in a reinforced membrane **700,** limited by the requirements of area **702** and providing suitable oxygen permeability.

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **721** wherein length **711** is between 2 millimeters (mm) and 72 mm and length **712** is between 2 mm and 72 mm. In an aspect, pattern **721** comprises a length **711** between 4 millimeters (mm) and 72 mm and length **712** is between 4 mm and 72 mm. In an aspect, pattern **721** comprises a length **711** between 9 millimeters (mm) and 72 mm and length **712** is between 9 mm and 72 mm. In an aspect, pattern **721** comprises a length **711** between 18 mm and 72 mm and length **712** is between 18 mm and 72 mm. In an aspect, pattern **721** comprises a length **711** between 27 millimeters (mm) and 72 mm and length **712** is between 27 mm and 72 mm. In an aspect, pattern **721** comprises a length **711** between 36 mm and 72 mm and length **712** is between 36 mm and 72 mm. In an aspect, pattern **721** comprises a length **711** between 63 mm and 72 mm and length **712** is between 63 mm and 72 mm. In other aspects, pattern **721** comprises a length **711** and a length **712** of 9 mm. In other aspects, pattern **721** comprises a length **711** and a length **712** of 18 mm. In other aspects, pattern **721** comprises a length **711** and a length **712** of 18 mm. In other aspects, pattern **721** comprises a length **711** and a length **712** of 27 mm. In other aspects, pattern **721** comprises a length **711** and a length **712** of 36 mm. In other aspects, pattern **721** comprises a length **711** and a length **712** of 63 mm. As used herein, pattern **721** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **722** wherein length **711** is between 2 millimeters (mm) and 72 mm. In an aspect, pattern **722** comprises a length **711** between 4 millimeters (mm) and 72 mm. In an aspect, pattern **722** comprises a length **711** between 9 millimeters (mm) and 72 mm. In an aspect, pattern **722** comprises a length **711** between 18 mm and 72 mm. In an aspect, pattern **722** comprises a length **711** between 27 millimeters (mm) and 72 mm. In an aspect, pattern **722** comprises a length **711** between 36 mm and 72 mm. In an aspect, pattern **722** comprises a length **711** between 63 mm and 72 mm. In other aspects, pattern **722** comprises a length **711** of 9 mm. In other aspects, pattern **722** comprises a length **711** of 18 mm. In other aspects, pattern **722** comprises a length **711** of 27 mm. In other aspects, pattern **722** comprises a length **711** of 36 mm. In other aspects, pattern **722** comprises a length **711** of 63 mm. As used herein, pattern **722** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **723** wherein the radius of length **718** is between 2 millimeters (mm) and 72 mm. In an aspect, pattern **723** comprises a radius of length **718** between 4 millimeters (mm) and 72 mm. In an aspect, pattern **723** comprises a radius of length **718** between 9 millimeters (mm) and 72 mm. In an aspect, pattern **723** comprises a radius of length **718** between 18 mm and 72 mm. In an aspect, pattern **723** comprises a radius of length **718** between 27 millimeters (mm) and 72 mm. In an aspect, pattern **723** comprises a radius of length **718** between 36 mm and 72 mm. In an aspect, pattern **723** comprises a radius of length **718** between 63 mm and 72 mm. In other aspects, pattern **723** comprises a radius of length **718** of 9 mm. In other aspects, pattern **723** comprises a radius of length **718** of 18 mm. In other aspects, pattern **723** comprises a radius of length **718** of 27 mm. In other aspects, pattern **723** comprises a radius of length **718** of 36 mm. In other aspects, pattern **723** comprises a radius of length **718** of 63 mm. As used herein, pattern **723** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **724** wherein length **711,** the distance between features **701,** is between 2 millimeters (mm) and 72 mm. In an aspect, pattern **724** comprises a length **711** between 4 millimeters (mm) and 72 mm. In an aspect, pattern **724** comprises a length **711** between 9 millimeters (mm) and 72 mm. In an aspect, pattern **724** comprises a length **711** between 18 mm and 72 mm. In an aspect, pattern **724** comprises a length **711** between 27 millimeters (mm) and 72 mm. In an aspect, pattern **724** comprises a length **711** between 36 mm and 72 mm. In an aspect, pattern **724** comprises a length **711** between 63 mm and 72 mm. In other aspects, pattern **724** comprises a length **711** of 9 mm. In other aspects, pattern **724** comprises a length **711** of 18 mm. In other aspects, pattern **724** comprises a length **711** of 27 mm. In other aspects, pattern **724** comprises a length **711** of 36 mm. In other aspects, pattern **722** comprises a length **711** of 63 mm. As used herein, pattern **724** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **725,** wherein length **711** is between 2 millimeters (mm) and 72 mm, wavelength **716** is between 5 mm and 200 mm, and amplitude **717** is between 5 mm and 72 mm. In an aspect, pattern **725** has a length **711** of between 4 millimeters (mm) and 72 mm, wavelength **716** of between 5 and 200 mm, and amplitude **717** of between 5 and 72 mm. In an aspect, pattern **725** has a length **711** of between 9 millimeters (mm) and 72 mm, wavelength **716** of between 5 and 200 mm, and amplitude **717** of between 5 and 72 mm. In an aspect, pattern **725** has a length **711** of between 27 millimeters (mm) and 72 mm, wavelength **716** of between 5 and 200 mm, and amplitude **717** of between 5 and 72 mm. In an aspect, pattern **725** has a length **711** of between 36 millimeters (mm) and 72 mm, wavelength **716** of between 5 and 200 mm, and amplitude **717** of between 5 and 72 mm. The a reinforced membrane **700** of pattern **725,** wherein length **711** is selected from the group consisting of 9 millimeters (mm), 18 mm, 27 mm, 36 mm, and 63 mm. As used herein, pattern **725** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **726** comprising a tile **710** wherein length **711** is between 2 millimeters (mm) and 72 mm and length **712** is between 2 mm and 72 mm. In an aspect, pattern **726** comprises a length **711** between 4 millimeters (mm) and 72 mm and length **712** is between 4 mm and 72 mm. In an aspect, pattern **726** comprises a length **711** between 9 millimeters (mm) and 72 mm and length **712** is between 9 mm and 72 mm. In an aspect, pattern **726** comprises a length **711** between 18 mm and 72 mm and length **712** is between 18 mm and 72 mm. In an aspect, pattern **726** comprises a length **711** between 27 millimeters (mm) and 72 mm and length **712** is between 27 mm and 72 mm. In an aspect, pattern **726** comprises a length **711** between 36 mm and 72 mm and length **712** is between 36 mm and 72 mm. In an aspect, pattern **726** comprises a length **711** between 63 mm and 72 mm and length **712** is between 63 mm and 72 mm. In other aspects, pattern **726** comprises a length **711** and a length **712** of 9 mm. In other aspects, pattern **726** comprises a length **711** and a length **712** of 18 mm. In other aspects, pattern **726** comprises a length **711** and a length **712** of 18 mm. In other aspects, pattern **726** comprises a length **711** and a length **712** of 27 mm. In other aspects, pattern **726** comprises a length **711** and a length **712** of 36 mm. In other aspects, pattern **726** comprises a length **711** and a length **712** of 63 mm. As used herein, pattern **726** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **727** wherein angle **719** is less than 60°, length **711** is between 2 millimeters (mm) and 72 mm and length **712** is between 2 mm and 72 mm. In an aspect, pattern **727** comprises a length **711** between 4 millimeters (mm) and 72 mm and length **712** is between 4 mm and 72 mm. In an aspect, pattern **727** comprises a length **711** between 9 millimeters (mm) and 72 mm and length **712** is between 9 mm and 72 mm. In an aspect, pattern **727** comprises a length **711** between 18 mm and 72 mm and length **712** is between 18 mm and 72 mm. In an aspect, pattern **727** comprises a length **711** between 27 millimeters (mm) and 72 mm and length **712** is between 27 mm and 72 mm. In an aspect, pattern **727** comprises a length **711** between 36 mm and 72 mm and length **712** is between 36 mm and 72 mm. In an aspect, pattern **727** comprises a length **711** between 63 mm and 72 mm and length **712** is between 63 mm and 72 mm. In other aspects, pattern **727** comprises a length **711** and a length **712** of 9 mm. In other aspects, pattern **727** comprises a length **711** and a length **712** of 18 mm. In other aspects, pattern **727** comprises a length **711** and a length **712** of 18 mm. In other aspects, pattern **727** comprises a length **711** and a length **712** of 27 mm. In other aspects, pattern **727** comprises a length **711** and a length **712** of 36 mm. In other aspects, pattern **727** comprises a length **711** and a length **712** of 63 mm. As used herein, pattern **727** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **728** wherein angle **719** is less than 90°, length **711** is between 2 millimeters (mm) and 72 mm and length **712** is between 2 mm and 72 mm. In an aspect, pattern **728** comprises a length **711** between 4 mm and 72 mm and length **712** is between 4 mm and 72 mm. In an aspect, pattern **728** comprises a length **711** between 9 mm and 72 mm and length **712** is between 9 mm and 72 mm. In an aspect, pattern **728** comprises a length **711** between 18 mm and 72 mm and length **712** is between 18 mm and 72 mm. In an aspect, pattern **728** comprises a length **711** between 27 mm and 72 mm and length **712** is between 27 mm and 72 mm. In an aspect, pattern **728** comprises a length **711** between 36 mm and 72 mm and length **712** is between 36 mm and 72 mm. In an aspect, pattern **728** comprises a length **711** between 63 mm and 72 mm and length **712** is between 63 mm and 72 mm. In other aspects, pattern **728** comprises a length **711** and a length **712** of 9 mm. In other aspects, pattern **728** comprises a length **711** and a length **712** of 18 mm. In other aspects, pattern **728** comprises a length **711** and a length **712** of 18 mm. In other aspects, pattern **728** comprises a length **711** and a length **712** of 27 mm. In other aspects, pattern **728** comprises a length **711** and a length **712** of 36 mm. In other aspects, pattern **728** comprises a length **711** and a length **712** of 63 mm. As used herein, pattern **728** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **728** wherein angle **719** is 90°, length **711** is between 2 millimeters (mm) and 72 mm and length **712** is between 2 mm and 72 mm. In an aspect, pattern **728** comprises a length **711** between 4 mm and 72 mm and length **712** is between 4 mm and 72 mm. In an aspect, pattern **728** comprises a length **711** between 9 mm and 72 mm and length **712** is between 9 mm and 72 mm. In an aspect, pattern **728** comprises a length **711** between 18 mm and 72 mm and length **712** is between 18 mm and 72 mm. In an aspect, pattern **728** comprises a length **711** between 27 mm and 72 mm and length **712** is between 27 mm and 72 mm. In an aspect, pattern **728** comprises a length **711** between 36 mm and 72 mm and length **712** is between 36 mm and 72 mm. In an aspect, pattern **728** comprises a length **711** between 63 mm and 72 mm and length **712** is between 63 mm and 72 mm. In other aspects, pattern **728** comprises a length **711** and a length **712** of 9 mm. In other aspects, pattern **728** comprises a length **711** and a length **712** of 18 mm. In other aspects, pattern **728** comprises a length **711** and a length **712** of 18 mm. In other aspects, pattern **728** comprises a length **711** and a length **712** of 27 mm. In other aspects, pattern **728** comprises a length **711** and a length **712** of 36 mm. In other aspects, pattern **728** comprises a length **711** and a length **712** of 63 mm. As used herein, pattern **728** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **729** wherein length **711** is between 2 millimeters (mm) and 72 mm and length **712** is between 2 mm and 72 mm. In an aspect, pattern **729** comprises a length **711** between 4 mm and 72 mm and length **712** is between 4 mm and 72 mm. In an aspect, pattern **729** comprises a length **711** between 9 mm and 72 mm and length **712** is between 9 mm and 72 mm. In an aspect, pattern **729** comprises a length **711** between 18 mm and 72 mm and length **712** is between 18 mm and 72 mm. In an aspect, pattern **729** comprises a length **711** between 27 mm and 72 mm and length **712** is between 27 mm and 72 mm. In an aspect, pattern **729** comprises a length **711** between 36 mm and 72 mm and length **712** is between 36 mm and 72 mm. In an aspect, pattern **729** comprises a length **711** between 63 mm and 72 mm and length **712** is between 63 mm and 72 mm. In other aspects, pattern **729** comprises a length **711** and a length **712** of 9 mm. In other aspects, pattern **729** comprises a length **711** and a length **712** of 18 mm. In other aspects, pattern **729** comprises a length **711** and a length **712** of 18 mm. In other aspects, pattern **729** comprises a length **711** and a length **712** of 27 mm. In other aspects, pattern **729** comprises a length **711** and a length **712** of 36 mm. In other aspects, pattern **729** comprises a length **711** and a length **712** of 63 mm. As used herein, pattern **729** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **730** comprising a tile **710** wherein length **711** is between 2 millimeters (mm) and 72 mm and length **712** is between 2 mm and 72 mm. In an aspect, pattern **730** comprises a length **711** between 4 millimeters (mm) and 72 mm and length **712** is between 4 mm and 72 mm. In an aspect, pattern **730** comprises a length **711** between 9 millimeters (mm) and 72 mm and length **712** is between 9 mm and 72 mm. In an aspect, pattern **730** comprises a length **711** between 18 mm and 72 mm and length **712** is between 18 mm and 72 mm. In an aspect, pattern **730** comprises a length **711** between 27 millimeters (mm) and 72 mm and length **712** is between 27 mm and 72 mm. In an aspect, pattern **730** comprises a length **711** between 36 mm and 72 mm and length **712** is between 36 mm and 72 mm. In an aspect, pattern **730** comprises a length **711** between 63 mm and 72 mm and length **712** is between 63 mm and 72 mm. In other aspects, pattern **730** comprises a length **711** and a length **712** of 9 mm. In other aspects, pattern **730** comprises a length **711** and a length **712** of 18 mm. In other aspects, pattern **730** comprises a length **711** and a length **712** of 18 mm. In other aspects, pattern **730** comprises a length **711** and a length **712** of 27 mm. In other aspects, pattern **730** comprises a length **711** and a length **712** of 36 mm. In other aspects, pattern **730** comprises a length **711** and a length **712** of 63 mm. As used herein, pattern **730** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **731** comprising a tile **710** wherein length **711** is between 2 millimeters (mm) and 72 mm and length **712** is between 2 mm and 72 mm. In an aspect, pattern **731** comprises a length **711** between 4 millimeters (mm) and 72 mm and length **712** is between 4 mm and 72 mm. In an aspect, pattern **731** comprises a length **711** between 9 millimeters (mm) and 72 mm and length **712** is between 9 mm and 72 mm. In an aspect, pattern **731** comprises a length **711** between 18 mm and 72 mm and length **712** is between 18 mm and 72 mm. In an aspect, pattern **731** comprises a length **711** between 27 millimeters (mm) and 72 mm and length **712** is between 27 mm and 72 mm. In an aspect, pattern **731** comprises a length **711** between 36 mm and 72 mm and length **712** is between 36 mm and 72 mm. In an aspect, pattern **731** comprises a length **711** between 63 mm and 72 mm and length **712** is between 63 mm and 72 mm. In other aspects, pattern **731** comprises a length **711** and a length **712** of 9 mm. In other aspects, pattern **731** comprises a length **711** and a length **712** of 18 mm. In other aspects, pattern **731** comprises a length **711** and a length **712** of 18 mm. In other aspects, pattern **731** comprises a length **711** and a length **712** of 27 mm. In other aspects, pattern **731** comprises a length **711** and a length **712** of 36 mm. In other aspects, pattern **731** comprises a length **711** and a length **712** of 63 mm. As used herein, pattern **731** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **732** comprising a tile **710** wherein length **711** is between 2 millimeters (mm) and 72 mm and length **712** is between 2 mm and 72 mm. In an aspect, pattern **732** comprises a length **711** between 4 millimeters (mm) and 72 mm and length **712** is between 4 mm and 72 mm. In an aspect, pattern **732** comprises a length **711** between 9 millimeters (mm) and 72 mm and length **712** is between 9 mm and 72 mm. In an aspect, pattern **732** comprises a length **711** between 18 mm and 72 mm and length **712** is between 18 mm and 72 mm. In an aspect, pattern **732** comprises a length **711** between 27 millimeters (mm) and 72 mm and length **712** is between 27 mm and 72 mm. In an aspect, pattern **732** comprises a length **711** between 36 mm and 72 mm and length **712** is between 36 mm and 72 mm. In an aspect, pattern **732** comprises a length **711** between 63 mm and 72 mm and length **712** is between 63 mm and 72 mm. In other aspects, pattern **732** comprises a length **711** and a length **712** of 9 mm. In other aspects, pattern **732** comprises a length **711** and a length **712** of 18 mm. In other aspects, pattern **732** comprises a length **711** and a length **712** of 18 mm. In other aspects, pattern **732** comprises a length **711** and a length **712** of 27 mm. In other aspects, pattern **732** comprises a length **711** and a length **712** of 36 mm. In other aspects, pattern **732** comprises a length **711** and a length **712** of 63 mm. As used herein, pattern **732** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** with a cross-section **760,** having a pattern **733.** Referring to Figure 8N, pattern 733 comprises a random arrangement of features **701** having a length **711** that is the average of the lengths of random features **701.** Accordingly, area **702** comprises the area of silicone membrane **113,** minus the product of the length **711,** the width of length **713,** and the number of features **701** (product **770).** As provided herein, area **702** is the difference between the total area of silicone membrane **113** and product **770.** In an aspect, length **711** of pattern **733** is between 2 millimeters (mm) and 72 mm. In an aspect, pattern **733** comprises a length **711** between 4 millimeters (mm) and 72 mm. In an aspect, pattern **733** comprises a length **711** between 9 millimeters (mm) and 72 mm. In an aspect, pattern **733** comprises a length **711** between 18 mm and 72 mm. In an aspect, pattern **733** comprises a length **711** between 27 millimeters (mm) and 72 mm. In an aspect, pattern **733** comprises a length **711** between 36 mm and 72 mm. In an aspect, pattern **733** comprises a length **711** between 63 mm and 72 mm. In other aspects, pattern **733** comprises a length **711** of 9 mm. In other aspects, pattern **733** comprises a length **711** of 18 mm. In other aspects, pattern **733** comprises a length **711** of 18 mm. In other aspects, pattern **733** comprises a length **711** of 27 mm. In other aspects, pattern **733** comprises a length **711** of 36 mm. In other aspects, pattern **733** comprises a length **711** of 63 mm. As used herein, pattern **733** can comprise a feature **701** with a cross-section **760** selected from the group consisting of **761** to **767.**

The present disclosure provides for, and includes, a reinforced membrane **700,** having silicone membrane **113** with an average thickness **703** of less than 100 x 10⁻⁶ M (µm) in area **702,** reinforced with features **701** having an average peak load of between 0.14 newton (N) and 1.9 N at a deflection of 12.5 mm. In an aspect, the average peak load is 0.4 N ± 0.05 N at a deflection of 12.5 mm.

The present disclosure provides for, and includes, a reinforced membrane **700,** having silicone membrane **113** with an average thickness **703** of less than 100 x 10⁻⁶ M (µm) in area **702,** reinforced with features **701** having an average peak load of between 0.14 newton (N) and 1.9 N at a deflection of 12.5 mm wherein the peak load is maintained for a period of 90 minutes when the load is applied at 1.2 hertz (Hz).

The present disclosure provides for, and includes, a reinforced membrane **700,** having silicone membrane **113** with an average thickness **703** of less than 100 x 10⁻⁶ M (µm) in area **702,** reinforced with features **701** wherein the average peak load is decreased between 150 and 350% compared to said silicone membrane **113** lacking said features **701.**

The present disclosure provides for, and includes, a reinforced membrane **700,** having silicone membrane **113** with an average thickness **703** of less than 100 x 10⁻⁶ M (µm) in area **702,** reinforced with features **701** wherein the average peak load is decreased between 200 and 350% compared to said silicone membrane **113** lacking said feature **701.**

The present disclosure provides for, and includes, methods for manufacturing reinforced membranes **700** having silicone membrane **113** with an average thickness **703** of less than 100 x 10⁻⁶ M (µm) in area **702,** reinforced with features **701** with a cross-section **760,** having a pattern **720.** In certain aspects, the methods of manufacture provide for the preparation of inner collapsible containers **102** comprising a reinforced membrane **700** in a single manufacturing step. The present disclosure provides for, and includes, for methods of manufacturing a reinforced silicone membrane **700** include knife coating, calendaring, compression molding, and injection molding. In an aspect, the method of preparing a silicone membrane **113** having a thickness of less than 100 x 10⁻⁶ M (µm) comprises knife coating.

The present disclosure provides for, and includes, an injection molded collapsible blood container **102** comprising a silicone membrane **700** comprising a silicone membrane **113** having an area **702** and an average thickness **703** of less than 100 x 10⁻⁶ M (µm) and a feature **701** on at least one side of said silicone membrane **113** having an average length **713** of between 100 µm and 10000 µm perpendicular to an average length **714** of between 20 µm and 5000 µm. In an aspect, the average thickness **703** of said area **702** is less than 50 µm thick. In another aspect, the average thickness **703** of said area **702** is less than 40 µm thick. In another aspect, the average thickness **703** of said area **702** is less than 30 µm thick. In another aspect, the average thickness **703** of said area **702** is less than 20 µm thick.

In another aspect, an injection molded collapsible blood container **102** comprising a silicone membrane **700** comprising a silicone membrane **113** having an area **702** and an average thickness **703** of between 5 and 100 µm thick. In some aspects, the area **702** has an average thickness **703** of between 5 and 75 µm thick. In additional aspects, the area **702** has an average thickness **703** of between 5 and 50 µm thick. In an aspect, the area **702** has an average thickness **703** of between 5 and 40 µm thick. In an aspect, the area **702** has an average thickness **703** of between 5 and 30 µm thick. In yet another aspect, the area **702** has an average thickness **703** of between 5 and 20 µm thick.

As provided for, and included in the present disclosure, an injection molded collapsible blood container **102** is sealed at the open end with Liquid Silicone Rubber (LSR), high consistency rubber (HCR), or a thermoplastic clip. In an aspect, the injection molded collapsible blood container **102** further includes at least one inlet/outlet 130. In certain aspects, at least two inlet/outlets 130 are included in the collapsible blood container **102.** In certain aspects, the one or more inlet/outlets **130** can be incorporated into the blow mold. In other aspects, the one or more inlet/outlets **130** can be incorporated into blow molded collapsible blood container **102** when the open end of the container is sealed.

The present disclosure provides for, and includes, an injection molded collapsible blood container **102** that empties without leakage within 2 minutes under an internal pressure of 50 kilopascals (kPa) above standard atmospheric pressure between two plates.

The present disclosure provides for, and includes, methods for manufacturing reinforced membranes **700** comprising preparing a silicone membrane **113** having a thickness of less than 100 x 10⁻⁶ M (µm), applying a raised feature **701** having an average length **713** of between 100 µm and 10000 µm perpendicular to an average length **714** of between 20 µm and 5000 µm to the surface of said silicone membrane **113,** and curing the resulting silicone membrane **700.** In an aspect, the method is a continuous process wherein the preparing a silicone membrane **113** having a thickness of less than 100 x 10⁻⁶ M (µm) and applying a raised feature **701** occurs in a single process step.

In an aspect, the manufacturing method comprises calendaring silicone between a first and second surface having at least one embossing feature **701.** In an aspect, the first surface comprises a roller having a recessed feature **701** arranged in a pattern **720** and the second surface is a conveyor. In an aspect, the method of calendaring comprises a first surface comprising a roller having a first recessed feature **701** arranged in a pattern **720** and a second surface comprising a roller having a second recessed feature **701** arranged in a pattern **720.**

### Examples:

### Example 1: Fabrication of an inner collapsible blood container 102, spot bonded reinforced silicone

A silicone bag is made from a pair of Wacker Silpuran^{®} silicone sheets about 7 inches square and 30 µm thick using Smooth-On Silpoxy^{®} adhesive to seal the perimeter with a piece of ¼" diameter silicone tubing bonded into the seam for fluid access to the lumen of the bag. The bag is reinforced by bonding two sheets of 4-mm square opening polypropylene mesh (Conwed #R3650) around the periphery on both sides of the bag using Smooth-On Silpoxy^{®} adhesive. The polypropylene mesh on one side of the bag is further bonded with four spots of adhesive about 5 x 10 mm spaced equally around the center of the bag; the polypropylene mesh on the other side of the bag is further bonded with five spots of adhesive about 5 mm diameter spaced equally around the center of the bag with one spot bond in the center. The bag is filled with water and drop tested from a height of about 3 feet multiple times with no leaks. Drop testing from about 5 feet resulted in bag rupture near the center of the side with 5 spot bonds.

### Example 2: Fabrication of reinforced silicone sheeting suitable for use in an inner collapsible blood container 102, partially cured sheet method

Silicone sheets having a thickness of about 25 µm are fabricated by mixing one part silicone LSR (liquid silicone rubber) part A with one part silicone LSR part B (NuSil MED10-6640) and diluted in 10 to 90% w/w xylene. The mixture is spread out and passed under a precision knife edge on a custom built knife coating machine to yield a sheet about 9 inches by 11 inches by about 50 µm thick before solvent evaporation. The sheet is partially cured by heating for about 3-5 minutes at 38-55°C (estimated) before placing a sheet of polyester mesh fabric (Mohawk Fabrics P/N 400485) onto the partially cured silicone sheet. The polyester mesh fabric is pressed into the sheet and placed back into an oven at 65-121°C for about 10 minutes to complete the curing of the LSR to yield a fabric reinforced silicone sheet about 9 inches by 11 inches and having an interstitial silicone thickness of about 25µm thick, not inclusive of the polyester mesh fabric reinforcement material. The polyester mesh fabric is adhered to the cured silicone sheet, but is not totally encapsulated by the silicone sheet. The one surface of the reinforced sheets has a matte finish suitable for contact with blood or blood products. Additional reinforced silicone sheets having thicknesses of about 13 µm and about 50 µm are fabricated using the partially cured sheet method.

### Example 3: Fabrication of an inner collapsible blood container 102, using reinforced silicone sheeting

To fabricate a blood bag from the fabric reinforced sheets, the fabric reinforced sheets are trimmed to about 9 inches square and a frame made of NuSil MED-4050 silicone HCR (High Consistency Rubber) is placed between the pair of fabric reinforced silicone sheets. Pressure is applied to this assembly while curing in an oven for about 30 minutes at 115-121 °C to yield an oxygen permeable blood bag that had internal dimensions of about 8 inches square. The oxygen permeable blood bag was able to reduce the blood oxygen content of a sample of LRpRBC to below 5% with agitation in the presence of oxygen sorbent within 3 hours.

Reinforced silicone collapsible blood containers **102** according to Figures 3 to 5 are prepared having the polyester mesh fabric prepared in Example 3 on the outside surface while the inside surface of the container is a matte finish silicone. The membrane 600 is joined together to form container **102** by a frame **120.** Each container includes a tube **121** for introducing and removing blood and other fluids. Containers **102** are tested by inflating with nitrogen to 1 psig and submerged in water for 30 seconds to test for leakage. Only containers that passed the leak test are used in the construction of an ORB.

### Example 4: Fabrication of reinforced silicone sheeting suitable for use in an inner collapsible blood container 102, fully cured sheet bonding method

A silicone sheet about 200 x 250 mm and about 20 µm thick (Wacker Silpuran^{®}) is bonded to a sheet of nylon fabric mesh by mixing one part silicone LSR (liquid silicone rubber) part A with one part silicone LSR part B (Wacker Silpuran^{®} 2030) mixed in about 10 to 90% w/w xylene, applying the silicone LSR mixture to the fabric and pressing the silicone sheet and fabric together with heat until the silicone LSR mixture is fully cured and the solvent is evaporated. A frame made of NuSil SIL2-5070 silicone HCR (High Consistency Rubber) is placed between the two fabric reinforced sheets, placed in a press under pressure and cured in an oven at about 115-121°C for about 10 minutes to yield a blood bag that is able to reduce the blood oxygen content of a unit of LRpRBC to below 5% with agitation in the presence of oxygen sorbent within 3 hours. Reinforced silicone collapsible blood containers **102** are prepared and tested as described in Example 3.

### Example 5: Drop test of 8"x8" reinforced inner collapsible blood containers

A reinforced silicone collapsible blood container **102** prepared according to Example 3 is assembled into an Oxygen Reduction Bag having a bed of Dessicare^{®} sorbent (Dessicare Part Number S1200B03, Dessicare, Inc., Reno, Nevada) in a Clearfoil^{®} Z barrier bag as described in the '130 Provisional Application. A total of 4 assemblies are tested after concluding deoxygenation tests. Blood is drained from the ORB assemblies after deoxygenation. The assemblies are then rinsed with saline and filled with 25% glycerol in water to simulate blood density (dyed blue to help visualize potential leaks). The assemblies are then dropped from a height of 6 feet onto a 12"x15" stainless steel plate in a large biohazard bag within a 16"x28"x13" high open topped cardboard box. Each ORB assembly is dropped one time only. The assemblies are dropped on the sorbent with the bag above the bed of sorbent, on the silicone bag with the bed of sorbent above the bag, on one corner of the assembly, and on the tube seal. All ORB assemblies pass the drop tests from 6 feet from all four drop configurations.

### Example 6: Oxygen Depletion of leukoreduced packed red blood cells using oxygen depletion devices with reinforced silicone collapsible blood containers having 14 um, 25 um, and 50 [an thick silicone membranes.

Oxygen depletion devices as described in the '130 Provisional were prepared using an outer receptacle 101 prepared from Clearfoil^{®} Z, a spacer 110 (McMaster Carr #9314T29, NJ McMaster Carr, Inc., Robbinsville, NJ), and a sorbent 103 provided in 15 sachets (Dessicare Part Number S1200B03, Dessicare, Inc., Reno, Nevada) using reinforced membranes 600 having thicknesses of 14 µm, 25 µm, and 50 µm as provided in Table 2.

For each test, four (4) each ORB Test Bags (2 x 146mL; 2x 110mL) are prepared having the indicated thickness and pooled type matched leukoreduced packed red blood cells (LR-pRBCs) are generated and oxygenated until the starting SO2 was >90%. A sample bag is sterile docked to the pool bag and a 3.5 mL sample is taken from the pool using a standard 5 ml syringe with a 16 gauge needle and tested for T=0 hemolysis levels using a HemoCue Plasma Analyzer. It is also tested on the ABL-90 for starting SO2 and pCO2 levels. About 146mL of pRBC product is transferred into two of the ORBs, and 110mL is transferred into the remaining two bags from the pool after sterile docking them. The source bag is disconnected from each ORB using a tube sealer and a sample bag is sterile docked to each ORB. ORBs are placed horizontally on a Helmer platelet shaker with the port and sampling bag on the left. Each ORB is deoxygenated on the platelet shaker for 3 hours. 1 ml samples are taken at T=0, 30, 60, 120 using a standard 1 ml syringe with a 16 gauge needle and are tested for SO2 and pCO2 levels on an ABL-90. A 3.5 ml sample is taken at T=180 min using a standard 5 ml syringe with a 16 gauge needle and is tested for hemolysis levels on the HemoCue Plasma Analyzer and for SO2 and pCO2 levels on an ABL-90. All samples are taken from the sampling bags sterile docked to each ORB. The results are summarized in Table 2.

**Table 2: Oxygen depletion using devices having inner blood containers prepared from reinforced silicone membranes**

| **Test bag number** # | **Silicone Membrane 113** | **Dimensions** | **Blood volume** | **Initial hematocrit** | **SO2 T=0** | **SO2 T=180** | **Rate constant** |
|---|---|---|---|---|---|---|---|
| | | **(inches)** | **(mL)** | **(%)** | **(%)** | **(%)** | **(min⁻¹)** |
| ORB1 | 13µm | 7.75x7.625 | 149.91 | 50.4 | 98.1 | 6.6 | -1.53E-02 |
| ORB2 | 13µm | 7.625x7.5 | 146.51 | 50.4 | 98.1 | 3.5 | -1.87E-02 |
| ORB3 | 13µm | 7.375x7.75 | 116.04 | 50.3 | 98.1 | 3.8 | -1.82E-02 |
| ORB 4 | 13µm | 7.5x7.25 | 110.47 | 50.4 | 98.2 | 4.3 | -1.74E-02 |
| ORB5 | 25µm | 6.5 x 7.5 | 149.15 | 49.6 | 98.4 | 5.6 | -1.66E-02 |
| ORB6 | 25µm | 6.5 x 7.5 | 149.81 | 49.6 | 98.7 | 5.6 | -1.60E-02 |
| ORB7 | 25µm | 6.5 x 7.5 | 115 | 56.2 | 98.6 | 5.1 | -1.67E-02 |
| ORB8 | 25µm | 6.5 x 7.5 | 115.66 | 49.3 | 98.8 | 6.7 | -1.55E-02 |
| ORB9 | 25µm | 10x16 | 115.66 | 59.1 | 96.8 | 6.3 | -1.54E-02 |
| ORB10 | 25µm | 10x16 | 115.66 | 59.0 | 97.1 | 8.8 | -1.42E-02 |
| ORB11 | 25µm | 10x16 | 115.66 | 58.5 | 97.5 | 10.3 | -1.40E-02 |
| ORB12 | 50µm | 7.75x7.625 | 149.34 | 56.6 | 98.6 | 9 | -1.48E-02 |
| ORB13 | 50µm | 7.75x7.625 | 149.15 | 56.2 | 100.1 | 10 | -1.29E-02 |
| ORB14 | 50µm | 7.75x7.625 | 111.79 | 56.5 | 100.1 | 4.5 | -1.50E-02 |
| ORB15 | 50µm | 7.75x7.625 | 110.94 | 56.3 | 99 | 7.8 | -1.43E-02 |

### Example 7: Automated reinforced silicone collapsible blood container manufacture from a roll of reinforced silicone sheet.

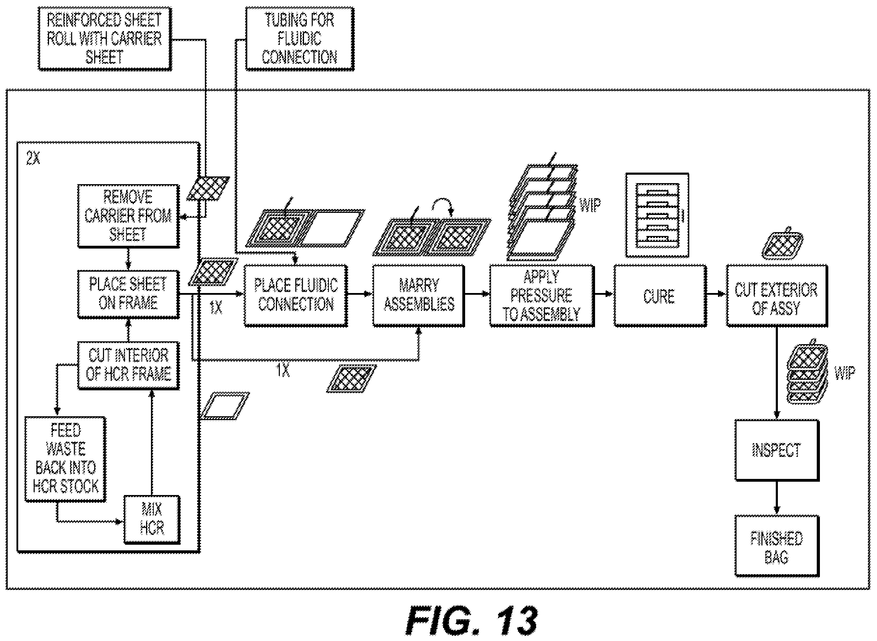

Referring to Figure 13, small and large scale reinforced silicone collapsible blood containers may be manufactured. As indicated as item 1, parts A and part B of a High Consistency Rubber (HCR) are mixed together in a two roll mill to create a homogenous high viscosity silicone sheet in an uncured state that is 0.25 mm - 0.2.5 mm thick. In item two, an HCR "frame" (frame **120** in Figures 3 to 5) is prepared by cutting the forming HCR sheet to size and desired interior shape using a steel rule, rotary style, or equivalent die. Excess material from the interior of the frame is returned to the uncured HCR stock as shown in item 3. Reinforced silicone sheets in roll form with a backing sheet are introduced and the backing removed leaving the silicone exposed (item 4). The reinforced silicone sheet is placed silicone side down, on the HCR frame from item 2 and shown as item 5. One or more subassemblies according to items 1 to 5 can be prepared. As shown in item 6, fluidic connections are introduced on one side of one frame/sheet subassembly from item 5. Suitable fluidic connections can be a silicone tube, a silicone over-molded barb fitting, or a barb fitting made of polycarbonate, nylon, polypropylene or other polymer. Plasma treatment may be optionally included to improve bonding between the fitting and HCR frame. A second frame/sheet subassembly from item 5 is placed on the onto the subassembly with fluidic connection from item 6, with the two HCR frames mated together as shown in item 7. As provided in item 8, pressure is applied to the assembly from step 7, using a press, rollers, or their equivalent at about 20 to 70 newtons (N) pressure, or an appropriate pressure to ensure contact of the assembly. The assembly is placed in an oven, a tunnel kiln, or their equivalent to cure, typically about 10 to 20 minutes at 115-130°C as shown in item 9. Following curing, the exterior of the assembly is cut to the desired final container shape, using a steel rule, a rotary die or their equivalent as shown in item 10. As shown in item 11, completed containers are inspected for imperfections and for leaks, for example using a pressure decay tool that measures a loss of pressure following filling the container with nitrogen. Multiple cycles of pressurizing, stabilizing, and measuring occur over a cycle time of about of 10-20 seconds are performed. Losses of pressure indicates a leak, thus a failed inspection.

### Example 8: Automated Manufacture of Reinforced Silicone Collapsible Blood Containers

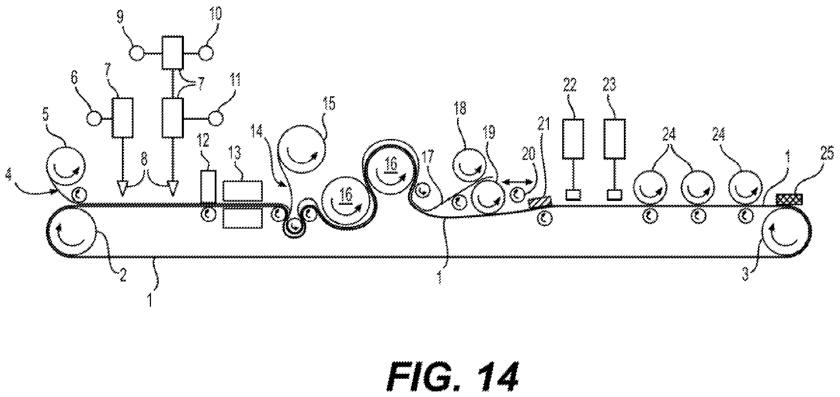

Referring to Figure 14, continuous process to form a reinforced silicone LSR (Liquid Silicone Rubber) film and further to fabricate a collapsible blood container from the film is provided. In an aspect, the process is driven by a conveyor belt.

The conveyor belt (1) has a drive roller (2) and a take-up roller (3). Optionally, a carrier film (4) may be used on top of the conveyor belt by feeding a polymer film, such as 0.006 inch thick polyester (Mylar^{®}) or PET (Polyethylene terephthalate) from a roll (5) onto the conveyor belt.

At the 1st process station the conveyor belt is treated by spraying a thin layer of silicone release agent using a pump (7) to dispense the material from a reservoir (6) through one or more spray nozzles (8) onto the conveyor belt. Optionally, the conveyor belt (or carrier film) may have a textured surface, wherein such texture is imparted to the silicone LSR film formed thereon and wherein such textured surface prevents the internal surfaces of the bag from sticking to each other and aids in filling the bag with blood.

At the 2nd process station a mixture of silicone LSR is formed by pumping the individual part A and part B of the LSR from their respective reservoirs (9,10), preferably through a mixing chamber (not shown). The uncured LSR mixture is then diluted by the addition of a suitable solvent, such as xylene, by pumping the solvated mixture through a pump (7) from a reservoir (11) and spraying the diluted mixture through one or more spray nozzles (8) onto the conveyor belt (or carrier film) to yield a thin film. The process variables (metering pumps, belt speed, etc.) are adjusted as needed to control the initial film thickness to about 50-75 µm. Optionally, the film thickness can be further controlled by passing under a knife edge (12) to reduce the film thickness to a desired thickness.

The conveyor belt passes through a heat tunnel (13) for a period of about 2-5 minutes at a temperature of about 125-200°F to partially cure the LSR and partially evaporate the solvent, yielding a LSR/xylene layer having a thickness of about 30-40 µm. Suitable means for heating include infrared lamps, quartz heating rods, and other resistively powered heating elements.

The conveyor belt passes under a roll (15) of polymer mesh (14), such as Mohawk Fabrics polyester P20D, which is spooled from the roll and fed onto the partially cured layer of LSR. Additional rollers may be employed to control the tension as needed to maintain the desired tension on the materials and contact between them.

The conveyor belt passes through a heated roller system (16) having a temperature of about 175-250°F. The number of heated rollers and their respective diameters and rotational speeds are configured and adjusted to match the timing of the previous processes, such that the combined process yields about 10 minutes of heating time to cure the LSR and remove the xylene solvent. The tension of the heated rollers is controlled and adjusted as needed to ensure the polyester fabric is in contact with the LSR layer. The cured polyester reinforced LSR composite film has a thickness of about 25 µm in the regions of LSR between the individual polyester reinforcing fibers.

At the 6th process station the cured reinforced LSR composite film (17) is separated from the conveyor belt (1) and cut into sheets by passing the film through a roller die (18) and a backing roller (19). Before depositing the cut sheets (21) back onto the conveyor belt, a roller mechanism (20) alternates the side of the sheet that is facing up when placed back onto the conveyor belt by engaging or disengaging the backing roller (19) as indicated by the arrow. If an optional carrier film is utilized, it is removed from the cut sheet at this time.

A silicone High Consistency Rubber (HCR) frame having an integrated tubing port is fed from a cassette feeder (22) and placed on top of the LSR sheets having the silicone surface facing up. The cut reinforced LSR sheet that has the polyester side facing up is lifted from the conveyor belt and placed on top of the sheet with the silicone HCR frame by a robotic arm (23) to yield a three-layer bag structure having two LSR sheets and one HCR frame.

The three-layer bag structures are then pressed together on the conveyor belt by a series of heated rollers (24), such that the distance between the heated rollers and the conveyor belt are maintained at a specified distance to provide slight compression to only the HCR frame, and to provide a curing cycle of about 10 minutes at 200-250°F. Optionally, the series of heated rollers may provide for incrementally increasing compression to ensure a leak-free seal of the LSR to HCR frame.

The cured three-layer collapsible blood containers (25) are transferred from the conveyor belt to a second conveyor belt system and second heat tunnel (not shown) for post-cure baking at about 230-250°F for about 120-240 minutes. To maintain balance with the previous process steps the second heat tunnel may utilize a cassette loader system to be able to accept multiple collapsible blood container structures before entering the second heat tunnel, wherein the number of collapsible blood container structures within a cassette tray balances the rate of the heat tunnel to the previous process steps.

The post-baked collapsible blood containers are transferred from the heat tunnel to a turntable (not shown) for cooling to ambient temperature (about 10 minutes) before removing the collapsible blood containers from the cassette holders. Alternatively, the cooled cassette holders are transferred with the collapsible blood containers for further assembly and packaging.

### Example 9: Fabrication of silicone reinforced silicone membranes 700 suitable for use in an inner collapsible blood container 102 using a compression method.

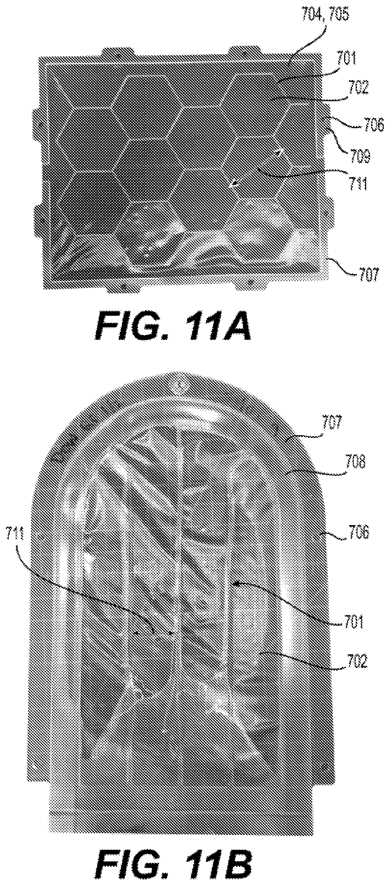

Plates are prepared having features **701** for compression molding of silicone reinforced silicone membranes **700** (Figure 11A). A compression molded silicone membrane **700** has reinforcing features **701** in between open areas of the silicone membrane **702** arranged in a pattern **722.** A membrane is made by depositing LSR on one plate and compressing it against the other plate set at a distance of 30µm apart to make a membrane 30µm thick. After compression, the resulting membrane is heated to 105°C for 10 minutes until set.

### Example 10: Application of reinforcement features to a preformed silicone membrane 113 suitable for use in an inner collapsible blood container 102.

A silicone membrane **113** is prepared using a knife coating technique as described in Example 2. After partial curing, 2-part LSR is prepared as described in Example 2 and applied with an 18 gauge plastic tip connected to a 30 CC syringe creating raised feature **701** arranged in a pattern **721.** The reinforcing raised features **701** have height **714,** base width **713,** and top width **715** and are separated by open areas **702** with an interstitial distance **711.** After LSR application, the reinforced membrane **700** is cured in an oven at 105°C for 10 minutes until set. A membrane **700** prepared according to this method is shown in Figure 10A.

### Example 11: Fabrication of an inner collapsible blood container 102 using silicone reinforced silicone membranes 700.

### A) LSR joining method

Two reinforced silicone membranes **700,** fabricated according to Example 10, are joined to prepare an inner collapsible blood container **102.** An integrated frame **120** is created by tracing the edge of a reinforced membrane **700** with 2-part LSR using a 14 gauge plastic tip connected to a 30 CC syringe on the blood contact side of the membrane. The features **701** are arranged on the exterior surface of the container **102.** Silicone tubing is placed on top of the frame in the desired location to form a fluidic connection **121** to the lumen of the container. A second reinforced silicone membrane **700** is joined to the first by the frame and the assembly is cured in an oven at 105°C for 10 minutes until set. A container **102** prepared according to this method is shown in Figure 10C.

### B) HCR joining method

Two reinforced silicone membranes **700** are joined by a die-cut frame **120** of HCR applied to the outer edge of a membrane. Space is left within the frame for placement of a silicone tube in the desired location to form a fluidic connection **121** to the lumen of the container. A second reinforced silicone membrane **700** is placed on top of the assembly, pressed to join, and cured in an oven at 105°C for 10 minutes until set.

### C) Injection molding method

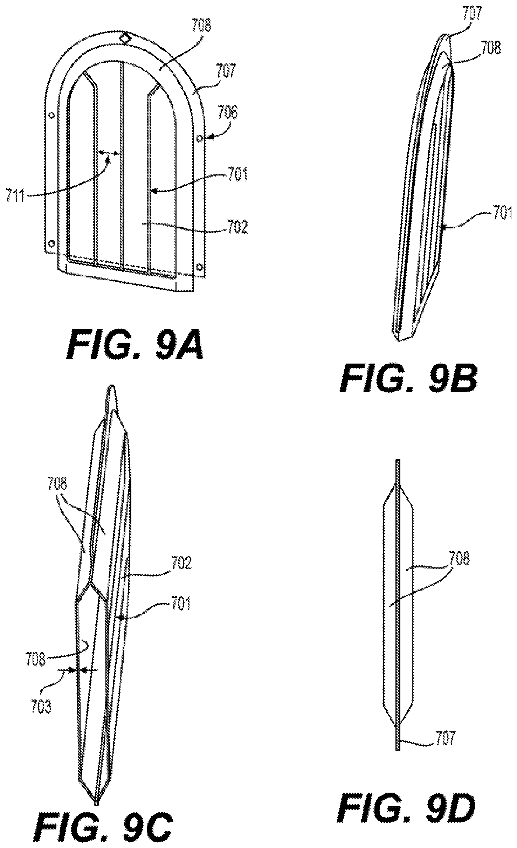

A collapsible blood container **102** is prepared using an injection mold prepared having a recessed feature **701*.** The mold is used to make a silicone container **102** comprised of silicone reinforced silicone membranes **700** with raised feature **701** open on one side in a single process (Figure 9 and 11B). After a removable core is inserted into the mold, LSR is prepared as described in Example 2 and injected into the mold under pressure. The silicone membranes have a thickness equivalent to the space between the cavity and the core. The container, as shown in Figure 11B, is cured in an oven at 105°C for 10 minutes until set. Silicone tubing is placed in the desired location to form a fluidic connection **121** to the lumen of the container and the open end is then sealed with LSR, HCR or a thermoplastic clip.

### Example 12: Testing peak force deflection over time.

Low-cycle fatigue testing is performed in accordance with a modified ASTM E606-12 method ("Standard Test Method for Strain-Controlled Fatigue Testing," Element Materials Technology) using a MTS Sintech Qtest compression testing machine ( S/N 022197, MTS Systems Corp.) in conjunction with an actuator (S/N P16-150-22-12-9) and software (Firgelli Technologies). Flexibility of silicone membranes is tested by mounting a 12.7 mm diameter membrane to a fixture having a central test area of 7.6mm in diameter. The test membrane is deflected 12.7 mm using a 2.54 mm spherical nozzle cycled at 1.2 hertz. The peak load at maximum deflection of each membrane is measured using a strain gauge attached to the spherical nozzle.

The peak load is determined every 5 minutes for 90 minutes during testing. The peak load values over the testing period are plotted and the average for each sample is obtained. Table 2 presents the results of 30 µm thick silicone membranes **113** as described in Example 10, fabric reinforced 25 µm silicone membranes **600** as described in Example 2, fabric reinforced 25 µm silicone membranes **600** that have been postcured and 30 µm silicone reinforced silicone membranes **700** as described in Example 11.

**Table 3: Average Peak load over 90 minutes**

| silicone membrane **113,** 30 µm | silicone membrane **600,** 25 µm | silicone membrane **600,** 25 µm postcured | silicone membrane **700**30 µm |
|---|---|---|---|
| 0.14 N | 6.82 N | 5.10 N | 0.39 N g |

### Example 13: Oxygen depletion testing using Oxygen depletion devices containing reinforced silicone membranes

ORBs are prepared for oxygen depletion testing as described in Example 6 with inner collapsible blood containers **102** composed of 30 µm silicone membranes **113,** fabric reinforced 25 µm silicone membranes **600,** internally reinforced 30 µm silicone membranes **700,** and externally reinforced 30 µm silicone membranes **700.**

Oxygen depletion testing is carried out as described in Example 6. All experiments begin with ≥90% SO₂ prior to transferring the blood to an ORB and placing on a platelet shaker for 90 minutes. The outer receptacle 101 headspace oxygen level is measured using a Mocon OpTech^{®} Platinum oxygen analyzer (MOCON, Inc., Minneapolis, MN). The kinetic rate and estimated final SO₂ concentration after 90 minutes is determined, the results are shown in Table 4.

**Table 4: Permeability of inner collapsible blood containers 102**

| | 30 µm silicone membrane, **113** | fabric reinforced 25 µm silicone membrane, **600** | internally reinforced 30 µm silicone membrane, **700** | externally reinforced 30 µm silicone membrane, **700** |
|---|---|---|---|---|
| Kinetic rate (x10⁻² min⁻¹) | -1.75 | -1.2 | -1.1 | -1.25 |
| Estimated % S0₂ after 90 min | 5.5% | 10% | 11.5% | 13.5% |
| *n* | 12 | 6 | 2 | 2 |

### Example 14: Permeability testing of reinforced silicone membranes.

Permeability to O₂ diffusion is tested using the MOCON permeability test system according to manufacturer's instructions (MOCON, Inc). In this system, a MOCON Optech^{®} O₂ Platinum analyzer is attached to a permeability test chamber and monitored using Optech^{®} O₂ Platinum software. The membrane sample is placed in the chamber with a Tyvek^{®} sheet on top and the chamber is sealed with the clamp. The tested is performed after the chamber is flushed with CO₂ until the partial pressure of O₂ stabilizes around 0.5-0.6 Torr.

Four membranes each of 30 µm silicone membranes **113,** fabric reinforced 25 µm silicone membranes **600,** and silicone reinforced 30 µm silicone membranes **700** are evaluated for 45 minutes. No detectable difference between the membranes are found. The permeability coefficient for 30 µm silicone membranes **113,** fabric reinforced 25 µm silicone membranes **600,** and silicone reinforced 30 µm silicone membranes **700** are all approximately 1.24 x 10⁻⁶ (ml*sec⁻¹*cm⁻²mmHg⁻¹).

While the invention has been described with reference to particular embodiments, it will be understood by those skilled in the art that various changes and modifications may be made without departing from the scope of the invention as defined in the appended claims.

## Claims

1. An oxygen depletion device (10) for depleting oxygen from blood prior to anaerobic storage comprising:
an outer receptacle (101) substantially impermeable to oxygen;
an inner collapsible blood container (102) comprising a fabric reinforced silicone membrane (600) and one or more chambers, wherein the fabric reinforced silicone membrane (600) comprises a silicone membrane (113) and a reinforcing fabric (620), and wherein the inner collapsible blood container (102) has a permeability to oxygen of greater than about 2.5 x 10⁻⁹ cm³ O₂ (STP)/((cm² s)*(cm Hg cm⁻¹));
a spacer (110) situated between the outer receptacle (101) and the inner collapsible blood container (102); and
an oxygen sorbent (103),
wherein the spacer (110) maintains a headspace defined by the inner collapsible blood container (102) and the outer receptacle (101), wherein the oxygen sorbent (103) is disposed in the headspace, and wherein the spacer (110) ensures efficient diffusion of oxygen from the surface of the silicone membrane (113) to the oxygen sorbent (103).

2. The oxygen depletion device of claim 1, wherein the reinforcing fabric (620) has discrete fibers (601) spaced at least about 0.1 millimeters (mm) to about 4 mm apart.

3. The oxygen depletion device of claim 1 or claim 2, wherein the oxygen sorbent (103) is also capable of carbon dioxide scavenging.

4. The oxygen depletion device of any one of claims 1-3, wherein the reinforcing fabric (620) is selected from the group consisting of polyester, nylon, and polyethylene.

5. The oxygen depletion device of any one of claims 1-4, wherein the fabric reinforced silicone membrane (600) has a thickness that is between 15 and 120 micrometers (µm).

6. The oxygen depletion device of any one of claims 1-5, wherein the inner collapsible blood container (102) is configured to have a surface area to volume ratio of at least 0.4 square centimeters per milliliter (cm²/ml) when filled with blood for depletion and enclosed within the outer receptable (101).

7. The oxygen depletion device of any one of claims 1-6, wherein the headspace has a volume of between 10 and 1000 milliliters (ml).

8. The oxygen depletion device of any one of claims 1-7, wherein the oxygen depletion device has a shelf life of at least 12 months after assembly.

9. The oxygen depletion device of any one of claims 1-8, wherein the inner collapsible blood container (102) further comprises flow baffles located internal or external to a blood contact area of the inner collapsible blood container (102), the flow baffles being configured to provide an increase in the turbulence inside the inner collapsible blood container (102) when agitated.

10. The oxygen depletion device of any one of claims 1-9, wherein the spacer (110) comprises one or more materials selected from the group consisting of a mesh, a molded mat, a woven mat, a non-woven mat, a strand veil, and a strand mat.

11. The oxygen depletion device of claim 2, wherein the fabric reinforced silicone membrane (600) further comprises a bonding layer (607) comprised of liquid silicone rubber (LSR).

## Patentansprüche

1. Sauerstoffabreicherungsvorrichtung (10) zum Abreichern von Sauerstoff aus Blut vor anaerober Lagerung, umfassend:
einen äußeren Behälter (101), der im Wesentlichen undurchlässig für Sauerstoff ist;
einen inneren kollabierbaren Blutbehälter (102), der eine gewebeverstärkte Silikonmembran (600) und eine oder mehrere Kammern umfasst, wobei die gewebeverstärkte Silikonmembran (600) eine Silikonmembran (113) und ein Verstärkungsgewebe (620) umfasst und wobei der innere kollabierbare Blutbehälter (102) eine Durchlässigkeit für Sauerstoff von größer als etwa 2,5 x 10⁻⁹ cm³ O₂ (STP)/((cm² s)*(m⁻¹ cm) Hg) aufweist;
einen Abstandshalter (110), der zwischen dem äußeren Behälter (101) und dem inneren kollabierbaren Blutbehälter (102) angeordnet ist; und
ein Sauerstoffsorbens (103),
wobei der Abstandshalter (110) einen Kopfraum aufrechthält, der durch den inneren kollabierbaren Blutbehälter (102) und den äußeren Behälter (101) definiert ist, wobei das Sauerstoffsorbens (103) in dem Kopfraum angeordnet ist und wobei der Abstandshalter (110) effiziente Diffusion von Sauerstoff von der Oberfläche der Silikonmembran (113) zu dem Sauerstoffsorbens (103) gewährleistet.

2. Sauerstoffabreicherungsvorrichtung nach Anspruch 1, wobei das Verstärkungsgewebe (620) diskrete Fasern (601) aufweist, die mindestens etwa 0,1 Millimeter (mm) bis etwa 4 mm voneinander beabstandet sind.

3. Sauerstoffabreicherungsvorrichtung nach Anspruch 1 oder Anspruch 2, wobei das Sauerstoffsorbens (103) ferner fähig ist, Kohlendioxid einzufangen.

4. Sauerstoffabreicherungsvorrichtung nach einem der Ansprüche 1-3, wobei das Verstärkungsgewebe (620) ausgewählt ist aus der Gruppe bestehend aus Polyester, Nylon und Polyethylen.

5. Sauerstoffabreicherungsvorrichtung nach einem der Ansprüche 1-4, wobei die gewebeverstärkte Silikonmembran (600) eine Dicke aufweist, die zwischen 15 und 120 Mikrometer (µm) beträgt.

6. Sauerstoffabreicherungsvorrichtung nach einem der Ansprüche 1-5, wobei der innere kollabierbare Blutbehälter (102) dafür gestaltet ist, ein Verhältnis von Oberfläche zu Volumen von mindestens 0,4 Quadratzentimeter pro Milliliter (cm²/ml) aufzuweisen, wenn er mit Blut zur Abreicherung gefüllt und in dem äußeren Behälter (101) eingeschlossen ist.

7. Sauerstoffabreicherungsvorrichtung nach einem der Ansprüche 1-6, wobei der Kopfraum ein Volumen zwischen 10 und 1000 Milliliter (ml) aufweist.

8. Sauerstoffabreicherungsvorrichtung nach einem der Ansprüche 1-7, wobei die Sauerstoffabreicherungsvorrichtung eine Haltbarkeit von mindestens 12 Monaten nach dem Zusammenbauen aufweist.

9. Sauerstoffabreicherungsvorrichtung nach einem der Ansprüche 1-8, wobei der innere kollabierbare Blutbehälter (102) ferner Strömungsleitelemente umfasst, die innerhalb oder außerhalb eines Blutkontaktbereichs des inneren kollabierbaren Blutbehälters (102) angeordnet sind, wobei die Strömungsleitelemente dafür gestaltet sind, wenn bewegt, eine Zunahme an Turbulenz innerhalb des inneren kollabierbaren Blutbehälters (102) bereitzustellen.

10. Sauerstoffabreicherungsvorrichtung nach einem der Ansprüche 1-9, wobei der Abstandshalter (110) ein oder mehrere Materialien ausgewählt aus der Gruppe bestehend aus einem Netz, einer geformten Matte, einer gewebten Matte, einer Vliesmatte, einem Fadenschleier und einer Fadenmatte umfasst.

11. Sauerstoffabreicherungsvorrichtung nach Anspruch 2, wobei die gewebeverstärkte Silikonmembran (600) ferner eine Bindeschicht (607) umfasst, die aus flüssigem Silikonkautschuk (LSR) besteht.

## Revendications

1. Dispositif d'appauvrissement en oxygène (10) pour appauvrir en oxygène le sang avant le stockage anaérobie, comprenant :
un récipient extérieur (101) sensiblement imperméable à l'oxygène ;
un récipient de sang repliable interne (102) comprenant une membrane en silicone renforcée par du tissu (600) et une ou plusieurs chambres, la membrane en silicone renforcée par du tissu (600) comprenant une membrane en silicone (113) et un tissu de renforcement (620), et le récipient de sang repliable interne (102) ayant une perméabilité à l'oxygène supérieure à environ 2,5 x 10⁻⁹ cm³ O₂ (STP)/((cm² s)*(cm Hg cm⁻¹)) ;
un espaceur (110) situé entre le réceptacle extérieur (101) et le récipient de sang repliable interne (102) ; et
un sorbant d'oxygène (103),
l'espaceur (110) maintenant un espace libre défini par le récipient de sang repliable interne (102) et le réceptacle extérieur (101), le sorbant d'oxygène (103) étant disposé dans l'espace libre, et l'espaceur (110) assurant une diffusion efficace d'oxygène de la surface de la membrane en silicone (113) vers le sorbant d'oxygène (103).

2. Dispositif d'appauvrissement en oxygène selon la revendication 1, le tissu de renforcement (620) comportant des fibres discrètes (601) espacées d'au moins environ 0,1 millimètre (mm) à environ 4 mm.

3. Dispositif d'appauvrissement en oxygène selon la revendication 1 ou la revendication 2, le sorbant d'oxygène (103) étant également capable de piéger le dioxyde de carbone.

4. Dispositif d'appauvrissement en oxygène selon l'une quelconque des revendications 1 à 3, le tissu de renforcement (620) étant choisi dans le groupe constitué par le polyester, le nylon et le polyéthylène.

5. Dispositif d'appauvrissement en oxygène selon l'une quelconque des revendications 1 à 4, la membrane en silicone renforcée par du tissu (600) ayant une épaisseur qui est comprise entre 15 et 120 micromètres (µm).

6. Dispositif d'appauvrissement en oxygène selon l'une quelconque des revendications 1 à 5, le récipient de sang repliable interne (102) étant configuré pour avoir un rapport surface/volume d'au moins 0,4 centimètre carré par millilitre (cm²/ml) lorsqu'il est rempli de sang pour appauvrissement et enfermé dans le récipient extérieur (101).

7. Dispositif d'appauvrissement en oxygène selon l'une quelconque des revendications 1 à 6, l'espace libre ayant un volume compris entre 10 et 1000 millilitres (ml).

8. Dispositif d'appauvrissement en oxygène selon l'une quelconque des revendications 1 à 7, le dispositif d'appauvrissement en oxygène ayant une durée de conservation d'au moins 12 mois après l'assemblage.

9. Dispositif d'appauvrissement en oxygène selon l'une quelconque des revendications 1 à 8, le récipient de sang repliable interne (102) comprenant en outre des déflecteurs d'écoulement situés à l'intérieur ou à l'extérieur d'une zone de contact avec le sang du récipient de sang repliable interne (102), les déflecteurs d'écoulement étant configurés pour fournir une augmentation de la turbulence à l'intérieur du récipient de sang repliable interne (102) lorsqu'ils sont agités.

10. Dispositif d'appauvrissement en oxygène selon l'une quelconque des revendications 1 à 9, l'espaceur (110) comprenant un ou plusieurs matériaux choisis dans le groupe constitué d'une maille, d'un mat moulé, d'un mat tissé, d'un mat non tissé, d'un voile à fils, et d'un mat à fils.

11. Dispositif d'appauvrissement en oxygène selon la revendication 2, la membrane en silicone renforcée par du tissu (600) comprenant en outre une couche de liaison (607) constituée de caoutchouc de silicone liquide (LSR).
